# EUROPEAN PATENT APPLICATION

(11) **EP 4 279 506 A1**
(43) Date of publication of application: **22.11.2023**
(21) Application number: 21919147.5
(22) Date of filing: 24.12.2021
(51) Int. Cl.: C07K 16/28, C12N 15/13, A61K 39/395, A61P 35/00

(54) **B7-H3 CHIMERIC ANTIGEN RECEPTOR-MODIFIED T CELL AND USE THEREOF**

(30) Priority: 13.01.2021 CN 202110042772
(71) Applicant: Persongen Biotherapeutics (Suzhou) Co., Ltd., Suzhou, Jiangsu 215000 (CN)
(72) Inventor: YANG, Lin, Suzhou, Jiangsu 215000 (CN); YOU, Fengtao, Suzhou, Jiangsu 215000 (CN); LI, Yafen, Suzhou, Jiangsu 215000 (CN)
(74) Representative: dompatent von Kreisler Selting Werner - Partnerschaft von Patent- und Rechtsanwälten mbB
(86) International application number: PCT/CN2021/141340
(87) International publication number: WO 2022/151960

(57) **Abstract**

Provided are a B7-H3 chimeric antigen receptor-modified T cell and the use thereof. Specifically, provided are an antibody targeting B7-H3, and a chimeric antigen receptor and an engineered immune cell prepared by means of using the antibody. Further provided are a method for preparing the antibody and the immune cell and the use thereof. The engineered immune cell of the present invention can effectively control the growth of tumor cells such as those of gastric cancer, osteosarcoma, breast cancer, neuroblastoma, pancreatic cancer and colon cancer, has no obvious toxicity and side effects in *in-vivo* experiments, has very good safety, and is promising as an effective immunotherapy for treating various solid tumors.

## Description

### TECHNICAL FIELD

The present invention relates to the field of biotechnology, and particularly to a B7-H3 chimeric antigen receptor-modified T cell and use thereof.

### BACKGROUND

The concept of Chimeric Antigen Receptor (CAR)-modified T cells for treating cancer is proposed in the end of 1980s. Through the development of thirty years, the CAR-T immunotherapy has achieved lots of remarkable accomplishment in the field of cancer therapy. In recent years, CAR-T cell makes a significant breakthrough in the treatment of blood tumors, in which the CAR-T therapy targeting CD19 antigen to treat B lymphocytic leukemia is most representative. In 2017, the United States FDA successively approved two therapies targeting juvenile relapsed and refractory chronic B lymphocytic leukemia (Kymriah) and adult relapsed and refractory large B cell lymphoma (Yeastar), respectively. A major research result obtained in the field of hematological tumor treatment by CAR-T cell therapy further excites the research and application thereof in the field of solid tumor treatment, but CAR-T cell therapy in the field of solid tumor treatment has not achieved an effect as satisfactory as in treatment of hematological tumors. One of the main reasons is that the antigens of the solid tumor are highly heterogeneous, in which targets are hard to be selected and it lacks an ideal treatment target like CD19.

B7-H3 molecule, also known as CD276 molecule, is a kind of recently discovered type I transmembrane glycoprotein, belonging to the B7 immune costimulatory and co-inhibitory globulin family. Human B7-H3 molecule has two subtypes, 2Ig-B7-H3 and 4Ig-B7-H3. Murine B7-H3 has only one subtype 2Ig-B7-H3, which is homologous with 88% of the amino acid sequence to human 2Ig-B7-H3 subtype. At present, the function of B7-H3 molecules is still going to be further elucidated, but the existing studies show that B7-H3 molecules have both immune activation and immunosuppressive effects. On one hand, the B7-H3 molecule can play an immunosuppressive effect by weakening the secretion of type I cytokine IFN-gamma, and many research results support B7-H3 to have a negative immunomodulatory effect in graft versus host disease, heart transplant rejection, airway inflammation and autoimmune encephalomyelitis model. On the other hand, B7-H3 is also found to exert a function of providing an immune co-stimulatory signal for T cells in autoimmune diseases *in vivo* and *in vitro.*

A large number of studies show that B7-H3 is overexpressed in various human tumors, including leukemia, gastric cancer, breast cancer, non-small cell lung cancer, ovarian cancer, osteosarcoma, neuroblastoma, pancreatic cancer, breast cancer and the like, but has only limited expression in normal tissue. At the same time, it is also reported to be expressed in important tumor microenvironment components such as tumor stem cells, tumor stromal cells and neovessels. The overexpression of B7-H3 can generally reduce the infiltration of tumor-associated lymphocytes in tumors and accelerate disease progression, and is generally closely related to the poor prognosis of cancers such as pancreatic ductal cancer, prostate cancer, ovarian cancer, lung cancer and transparent cell renal cancer.

Gastric cancer is a very common malignant tumor in the world, which is listed as the fifth most common cancer and the third most common cause of cancer death. Gastric cancer patients have no obvious symptoms in the early stage, and are mostly diagnosed in late stage. In addition, GC cells are easy to transfer and proliferate, and frequently relapse after surgery. In the past decades, although the treatment of gastric cancer is improved, the median overall survival (mOS) is only 6.6 to 18.4 months. FDA has approved trastuzumab (anti-ERBB2/HER2) and ramycin monoclonal antibodies (anti-KDR/VEGFR2) to treat advanced or metastatic gastric cancer, but the survival rate is still not ideal and drug resistance occurs. B7-H3 molecules are reported to be highly expressed in tumor tissues of gastric cancer patients, and the expression of B7-H3 molecules is closely related to metastasis of gastric cancer cells and poor prognosis of patients.

Triple-negative breast cancer is a class of refractory breast cancer, characterized in non-expression of progestogen receptors, estrogen receptors and HER2 antigens, which develops more commonly in young people, African Americans, Hispanics, or those with BRCA1 gene mutations, and accounts for approximately 10-15% of diagnosed breast cancer cases. Due to the lack of these three types of receptors, it can not be effectively treated by general treatment methods, such as drugs targeting estrogen receptors, progestogen receptors, and HER-2 antigens. At present, combined therapy of methods such as surgery, chemotherapy, radiotherapy and the like is still an effective choice for treating triple-negative breast cancer. The five-year survival rate of triple-negative breast cancer is 83%, lower than other forms of breast cancer, and the triple-negative breast cancer is more prone to recur after treatment than other types of breast cancer. Studies have reported that B7-H3 antigens are highly expressed in metastatic and malignant breast cancer tissues.

Osteosarcoma is also called osteogenic sarcoma, which is a tumor commonly developing in children and teenagers, accounting for approximately 3% of pediatric tumors. Osteosarcoma is typically caused by the canceration of new bone growth cells, and thus, in children and teenagers, it typically occurs at the end of the long bone that is fastest in bone growth. Currently, the treatment methods for osteosarcoma mainly include operations, radiotherapy and chemotherapy. The cure rate for osteosarcoma patients without metastasis is usually higher, and about three-thirds of the patients can be cured. But the survival rate of patients with already transferred osteosarcoma cells is only 30%. Studies have shown that B7-H3 molecules are highly expressed in tumor tissues of 90% or more osteosarcoma patients, and the expression of B7-H3 is closely related to the recurrence of osteosarcoma and the survival of patients.

Pancreatic cancer refers to a malignant tumor that develops in the pancreas, and is divided into exocrine malignant tumor of the pancreas and endocrine malignant tumor of the pancreas. The exocrine malignant tumors of the pancreas are mainly adenocarcinoma derived from pancreatic ductal epithelium, accounting for about 90% or more of pancreatic cancers. Therefore, the pancreatic cancer is generally referred to a pancreatic ductal epithelial cancer. Other relatively common exocrine malignant tumors also include pancreatic acinar cell carcinoma and cystadenocarcinoma of the pancreas. Additionally, a portion of pancreatic cancers are derived from neuroendocrine cells, such as malignant insulin tumors and gastrin tumors, and may have symptoms caused by a corresponding increase in hormone. The malignant degree of pancreatic cancer is very high, accounting for about 1% -2% of the whole malignant tumor, and has a trend of increasing year by year. In pancreatic cancer cells, B7-H3 increases IL-8 and VEGF expression by activating a TLR4/NF-kb signal pathway, thereby promoting proliferation, migration and infiltration of pancreatic cancer cells. Therefore, B7-H3 is an ideal target for targeting treatment of pancreatic cancer.

Neuroblastoma is a malignant tumor derived from sympathetic nerves, mainly composed of undifferentiated neuroblastoma, and mainly develops in children. Nearly half of neuroblastoma occurs in infants under 2 years old, with relatively complex conditions, relatively clear tumor boundary, relatively hard texture, and normally with bleeding, necrosis or cystic deformation. The clinical manifestations are relatively complex. It can occur at different parts of a human, and have different performances according to different parts. Neuroblastoma mainly occurs in the thoracic cavity, the abdominal cavity and the neck, of which the treatment means are complex, and the prognosis is also different. In general, the malignant degree of neuroblastoma is very high, and it is difficult to cure. Studies have found that the neuroblastoma cell strain highly expresses B7-H3 molecules.

Colon cancer is a common digestive tract malignant tumor, which accounts for the second largest number of gastrointestinal tumors. The common site is rectum and the junction of rectum and sigmoid colon, accounting for 60%, and it usually occurs after the age of 40, and the male to female ratiois 2: 1. The onset of colon cancer is mainly related to a diet of high fat and low cellulose. The chronic inflammation of the colon causes a higher incidence of colon cancer than the general population. The incidence of colon cancer in those with colon polyp is 5 times higher than in those without colon polyp. In those having familial multiple colon sarcoma, the incidence of canceration is higher. Genetic factors may also participate in the onset of colon cancer. The early symptoms of colon cancer are obvious, and the middle and late symptoms can appear as abdominal distension, dyspepsia, and then changes in defecation habit, abdominal pain, mucous stool or sticky blood stool may occur. After tumor ulceration, blood loss and toxin absorption, anemia, low heat, weakness, emaciation, lower limb edema and other symptoms often occur. Studies have shown that B7-H3 is also highly expressed in colon cancer cell lines.

Due to the above features of B7-H3 molecule, it has become one of the popular targets for tumor immunotherapy in recent years. The current two monoclonal antibodies 8H9 and MGA271 targeting B7-H3 have been clinically proved to be safe and effective. However, the study of the existing B7-H3 monoclonal antibodies, especially B7-H3 chimeric antigen receptor-modified T cells also has many disadvantages. It is necessary to develop a new B7-H3 monoclonal antibody and related CAR-T cells and uses thereof in the art.

### SUMMARY OF THE INVENTION

The purpose of the invention is to provide a B7-H3 chimeric antigen receptor-modified T cell and use thereof.

In the first aspect of the present invention, it provides an antibody targeting B7-H3, which comprises a heavy chain variable region and/or a light chain variable region;
wherein the heavy chain variable region comprises the following complementarity determining regions (CDRs):
a CDR1 as shown in SEQ ID NO: 2;
a CDR2 as shown in SEQ ID NO: 3; and
a CDR3 as shown in SEQ ID NO: 4; and
the light chain variable region comprises the following complementarity determining regions (CDRs):
   a CDR1' as shown in SEQ ID NO: 6;
   a CDR2' as shown in SEQ ID NO: 7; and
   a CDR3' as shown in SEQ ID NO: 8.

In another preferred embodiment, the amino acid sequence of the heavy chain variable region of the antibody is shown in SEQ ID NO: 1, 9 or 11.

In another preferred embodiment, the amino acid sequence of the light chain variable region of the antibody is shown in SEQ ID NO: 5, 10 or 12.

In another preferred embodiment, the amino acid sequence of the heavy chain variable region of the antibody is shown in SEQ ID NO: 1, and the amino acid sequence of the light chain variable region of the antibody is shown in SEQ ID NO: 5.

In another preferred embodiment, the amino acid sequence of the heavy chain variable region of the antibody is shown in SEQ ID NO: 9, and the amino acid sequence of the light chain variable region of the antibody is shown in SEQ ID NO: 10.

In another preferred embodiment, the amino acid sequence of the heavy chain variable region of the antibody is shown in SEQ ID NO: 11, and the amino acid sequence of the light chain variable region of the antibody is shown in SEQ ID NO: 12.

In another preferred embodiment, any one of the above amino acid sequences further includes a derivative sequence which is optionally added, deleted, modified, and/or substituted with at least one amino acid, and can retain the binding affinity to B7-H3 .

In another preferred embodiment, the number of added, deleted, modified and/or substituted amino acids is 1-5 (such as 1-3, preferably 1-2, more preferably 1).

In another preferred embodiment, the derivative sequence that is added, deleted, modified and/or substituted with at least one amino acid and can retain B7-H3 binding affinity is an amino acid sequence with homology or sequence identity of at least 80%, 85%, 90%, 91%, 92%, 93%, 94%, 95%, 96%, 97%, 98%, or 99%.

In another preferred embodiment, the antibody further comprises a heavy chain constant region and/or a light chain constant region.

In another preferred embodiment, the heavy chain constant region is of human origin or murine origin.

In another preferred embodiment, the heavy chain constant region is a human antibody heavy chain IgG1 constant region.

In another preferred embodiment, the light chain constant region is of human origin or murine origin.

In another preferred embodiment, the light chain constant region is a human antibody light chain kappa constant region.

In another preferred embodiment, the antibody is selected from the group consisting of an animal-derived antibody, chimeric antibody, humanized antibody, fully human antibody, and a combination thereof.

In another preferred embodiment, the ratio (Z1/Z0) of the immunogenicity of the chimeric antibody in humans (Z1) and the immunogenicity of non-chimeric antibody (such as murine antibodies) in humans (Z0) is 0-0.5, preferably 0-0.2, more preferably 0-0.05 (e.g., 0.001-0.05).

In another preferred embodiment, the antibody is a double-chain antibody or a single-chain antibody.

In another preferred embodiment, the antibody is a full-length protein of an antibody, or an antigen binding fragment.

In another preferred embodiment, the antibody is a bispecific antibody or a multispecific antibody.

In the second aspect of the present invention, it provides a scFv targeting B7-H3, wherein the heavy chain variable region of the scFv comprises the following complementarity determining regions (CDRs):
a CDR1 as shown in SEQ ID NO: 2;
a CDR2 as shown in SEQ ID NO: 3; and
a CDR3 as shown in SEQ ID NO: 4;
and the light chain variable region of the scFv comprises the following complementarity determining regions (CDRs):
   a CDR1' as shown in SEQ ID NO: 6;
   a CDR2' as shown in SEQ ID NO: 7; and
   a CDR3' as shown in SEQ ID NO: 8.

In another preferred embodiment, the scFv further comprises a linking peptide between the heavy chain constant region and the light chain constant region.

In another preferred embodiment, the linking peptide has 1-4 continuous sequences shown in SEQ ID NO: 17 (GGGGS), preferably 3-4, more preferably 4.

In another preferred embodiment, the scFv is shown in the following Formula A or B:

V_{H}-V_{L}, (A); V_{L}-V_{H}, (B)

wherein, V_{H} represents the heavy chain variable region of the antibody; V_{L} represents the light chain variable region of the antibody; and "-" represents a linking peptide or a peptid bond.

In another preferred embodiment, the scFv is shown in Formula A (V_{H}-V_{L}).

In another preferred embodiment, the amino acid sequence of the scFv is shown in SEQ ID NO: 13, 14 or 18.

In another aspect of the present invention, it provides a recombinant protein comprising:
(i) the antibody according to the first aspect of the present invention; and
(ii) an optional tag sequence to assist expression and/or purification.

In another preferred embodiment, the tag sequence comprises a 6His tag.

In another preferred embodiment, the recombinant protein (or polypeptide) comprises a fusion protein.

In another preferred embodiment, the recombinant protein is a monomer, dimer, or multimer.

In the third aspect of the present invention, it provides a chimeric antigen receptor (CAR) comprising the scFv according to the second aspect of the present invention.

In another preferred embodiment, the antigen binding domain of the CAR comprises an antibody heavy chain variable region shown in SEQ ID NO: 1, 9, or 11, and an antibody light chain variable region shown in SEQ ID NO: 5, 10, or 12.

In another preferred embodiment, the CAR has a structure shown in the following Formula I:

L-scFv-H-TM-C-CD3ζ (I)

wherein,
each "-" is independently a linking peptide or peptide bond;
L is none or a signal peptide sequence;
scFv is a scFv targeting B7-H3;
H is an optional hinge region;
TM is a transmembrane domain;
C is a co-stimulatory signaling molecule;
CD3ζ is a CD3ζ cytoplasmic signal transduction sequence.

In another preferred embodiment, L is a signal peptide of a protein selected from the group consisting of EF1, CD8, GM-CSF, CD4, CD137, and a combination thereof.

In another preferred embodiment, L is a signal peptide derived from EF1.

In another preferred embodiment, the scFv is shown in the following Formula A or B:

V_{H}-V_{L}, (A); V_{L}-V_{H}, (B)

wherein, V_{H} represents the heavy chain variable region of the antibody; V_{L} represents the light chain variable region of the antibody; and "-" represents a linking peptide or a peptid bond.

In another preferred embodiment, the scFv is shown in Formula A (V_{H}-V_{L}).

In another preferred embodiment, H is a hinge of a protein selected from the group consisting of CD8, CD28, CD137, Fc, and a combination thereof.

In another preferred embodiment, H is a hinge derived from CD8 or Fc.

In another preferred embodiment, TM is a transmembrane region of a protein selected from the group consisting of CD28, CD3 epsilon, CD45, CD4, CD5, CD8, CD9, CD16, CD22, CD33, CD37, CD64, CD80, CD86, CD134, CD137, CD154, and a combination thereof.

In another preferred embodiment, TM is a transmembrane region derived from CD8.

In another preferred embodiment, TM is a transmembrane region derived from ICOS.

In another preferred embodiment, C is a co-stimulatory signaling molecule of a protein selected from the group consisting of OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2, and a combination thereof.

In another preferred embodiment, C is a co-stimulatory signaling molecule derived from 4-1BB.

In another preferred embodiment, C is a co-stimulatory signaling molecule derived from ICOS and 4-1BB.

In another preferred embodiment, the CAR further comprises a cell suicide element (preferably at C-terminus).

In another preferred embodiment, the CAR is linked with the cell suicide element via a self-cleaving element.

In another preferred embodiment, the cell suicide element is linked with CD3ζ of the CAR via a T2A.

In another preferred embodiment, the cell suicide element is selected from the group consisting of HSV-TK, iCasp9, ΔCD20, mTMPK, ΔCD19, RQR8, EGFRt, and a combination thereof.

In another preferred embodiment, the cell suicide element is tEGFR.

In another preferred embodiment, the amino acid sequence of the tEGFR is shown in SEQ ID NO: 15.

In another preferred embodiment, the amino acid sequence of the CAR is shown in SEQ ID NO: 16.

In another preferred embodiment, H is a hinge derived from CD8 or Fc, TM is a transmembrane region derived from ICOS, and C is a co-stimulatory signaling molecule derived from ICOS and 4-1BB.

In another preferred embodiment, the amino acid sequence of the CAR is shown in SEQ ID NO: 19.

In the fourth aspect of the present invention, it provides a polynucleotide, which encodes a polypeptide selected from the group consisting of:
(1) the antibody according to the first aspect of the present invention;
(2) the scFv according to the second aspect of the present invention; and
(3) the chimeric antigen receptor according to the third aspect of the present invention.

In the fifth aspect of the present invention, it provides a vector, which contains the polynucleotide according to the fourth aspect of the present invention.

In another preferred embodiment, the vector includes: a bacterial plasmid, phage, yeast plasmid, plant cell virus, mammalian cell virus such as adenovirus, retrovirus, or other vectors.

In another preferred embodiment, the vector includes a lentiviral vector.

In the sixth aspect of the present invention, it provides a genetically engineered host cell, which comprises the vector according to the fifth aspect of the present invention, or has the exogenous polynucleotide according to the fourth aspect of the present invention integrated into the genome, or expresses the antibody according to the first aspect of the present invention, or expresses the scFv according to the second aspect of the present invention, or expresses the chimeric antigen receptor according to the third aspect of the present invention.

In another preferred embodiment, the cell is an isolated cell, and/or the cell is a genetically engineered cell.

In another preferred embodiment, the immune cell is derived from human or a non-human mammal (such as a mouse).

In another preferred embodiment, the cell includes a T cell and an NK cell.

In another preferred embodiment, the host cell is an engineered immune cell.

In another preferred embodiment, the immune cell expresses an exogenous cell suicide element.

In another preferred embodiment, the CAR and the cell suicide element are co-expressed in the immune cell.

In another preferred embodiment, the engineered immune cell includes a T cell or an NK cell, and preferably is (i) a chimeric antigen receptor T cell (CAR-T cell); or (ii) a chimeric antigen receptor NK cell (CAR-NK cell).

In the seventh aspect of the present invention, it provides an antibody conjugate, which comprises:
(a) an antibody moiety, which is selected from the group consisting of the antibody according to the first aspect of the present invention; and
(b) a coupling moiety coupled to the antibody moiety, which is selected from the group consisting of a detectable label, a drug, a toxin, a cytokine, a radionuclide, an enzyme, and a combination thereof.

In another preferred embodiment, the antibody moiety and the coupling moiety are coupled through a chemical bond or a linker.

In another preferred embodiment, the antibody conjugate is an antibody-drug conjugate.

In the eighth aspect of the present invention, it provides a method for preparing an engineered cell expressing the antibody of the first aspect of the present invention, the scFv of the second aspect of the present invention or the chimeric antigen receptor of the third aspect of the present invention, which comprises a step of: transducing the polynucleotide of the fourth aspect of the present invention or the vector of the fifth aspect of the present invention into an host cell, thereby obtaining the engineered cell.

In another preferred embodiment, the method further comprises a step of: transducing a cassette for expressing a cell suicide element into the immune cell.

In another preferred embodiment, the method further comprises a step of detecting the function and effectiveness of the obtained engineered cell.

In the ninth aspect of the present invention, it provides a pharmaceutical composition comprising the antibody according to the first aspect of the present invention, the scFv protein according to the second aspect of the present invention, the CAR according to the third aspect of the present invention, the vector according to the fifth aspect of the present invention, the host cell according to the sixth aspect of the present invention, the antibody conjugate according to the seventh aspect of the present invention, and a pharmaceutically acceptable carrier, diluent or excipient.

In another preferred embodiment, the pharmaceutical composition is a liquid preparation.

In another preferred embodiment, the pharmaceutical composition is an injection.

In another preferred embodiment, the pharmaceutical composition further comprises a second active ingredient of anti-tumor, which preferably comprises a second antibody, or a chemotherapeutic agent.

In another preferred embodiment, the chemotherapeutic agent is selected from the group consisting of Docetaxel, carboplatin, and a combination thereof.

In the tenth aspect of the present invention, it provides a use of an active ingredient selected from the group consisting of: the antibody according to the first aspect of the present invention, the scFv according to the second aspect of the present invention, the CAR according to the third aspect of the present invention, the vector according to the fifth aspect of the present invention, the host cell according to the sixth aspect of the present invention, or the antibody conjugate according to the seventh aspect of the present invention, for (a) preparation of a diagnostic reagent or kit; and/or (b) preparation of a medicament or formulation for the prevention and/or treatment of a tumor or cancer.

In another preferred embodiment, the tumor is a B7-H3-positive tumor.

In another preferred embodiment, the tumor is selected from the group consisting of: hematological tumors, solid tumors, and a combination thereof.

In another preferred embodiment, the hematological tumor is selected from the group consisting of acute myeloid leukemia (AML), multiple myeloma (MM), chronic lymphocytic leukemia (CLL), acute lymphocytic leukemia (ALL), diffuse large B cell lymphoma (DLBCL), and a combination thereof.

In another preferred embodiment, the solid tumor is selected from the group consisting of gastric cancer, gastric cancer peritoneal metastasis, liver cancer, kidney tumor, lung cancer, small intestinal cancer, bone cancer, prostate cancer, colorectal cancer, breast cancer, colorectal cancer, cervical cancer, ovarian cancer, lymphoma, nasopharyngeal cancer, adrenal tumor, bladder tumor, non-small cell lung cancer (NSCLC), brain glioma, endometrial cancer, testicular cancer, colorectal cancer, urinary tract tumor, thyroid cancer, and a combination thereof.

In another preferred embodiment, the tumor is a gastric cancer, a breast cancer, a osteosarcoma, a neuroblastoma, a pancreatic caner, or a colorectal cancer.

In another preferred embodiment, the diagnostic reagent is a detection slide or detection plate.

In another preferred embodiment, the diagnostic reagent or kit is used for:
(1) detection of B7-H3 protein in a sample; and/or
(2) detection of endogenous B7-H3 protein in tumor cells; and/or
(3) detection of tumor cells expressing B7-H3 protein.

In the eleventh aspect of the present invention, it provides a method for detection (including diagnostic or non-diagnostic detection) of B7-H3 protein in a sample, wherein the method comprises the steps of:
(1) contacting the sample with the antibody according to the first aspect of the present invention, the scFv according to the second aspect of the present invention, or the antibody conjugate according to the seventh aspect of the present invention *in vitro*; and
(2) detecting whether an antigen-antibody complex is formed, wherein the formation of the complex indicates the presence of B7-H3 protein in the sample.

In the twelfth aspect of the present invention, it provides a detection plate, wherein the detection plate comprises: a substrate (support plate) and a detection strip, wherein the detection strip comprises the antibody according to the first aspect of the present invention, the recombinant protein according to the second aspect of the present invention, or the antibody conjugate according to the seventh aspect of the present invention, or a combination thereof.

In the thirteenth aspect of the present invention, it provides a kit, which comprises:
(1) a first container, which contains the antibody according to the first aspect of the present invention; and/or
(2) a second container, which contains a secondary antibody against the antibody according to the first aspect of the present invention;

alternatively,
the kit comprises the detection plate according to the twelfth aspect of the present invention.

In the fourteenth aspect of the present invention, it provides a method for preparing a recombinant polypeptide, wherein the method comprises:
(a) culturing the host cell according to the sixth aspect of the present invention under conditions suitable for expression;
(b) isolating a recombinant polypeptide from the culture, wherein the recombinant polypeptide is the antibody according to the first aspect of the present invention or the scFv according to the second aspect of the present invention.

In the fifteenth aspect of the present invention, it provides a method for treating diseases, comprising administering to a subject in need an effective amount of the antibody according to the first aspect of the present invention, or the scFv according to the second aspect of the present invention, or the antibody conjugate according to the seventh aspect of the present invention, or the host cell according to the sixth aspect of the present invention, or the pharmaceutical composition according to the ninth aspect of the present invention, or a combination thereof.

In another preferred embodiment, the disease is a B7-H3-positive tumor.

It should be understood that, within the scope of the present invention, the technical features specifically described above and below (such as the Examples) can be combined with each other, thereby constituting a new or preferred technical solution which needs not be described one by one.

### Description of Figure

Figure 1A shows an expression vector profile of the light chain and heavy chain of humanized CD276 antibody.
Figure 1B shows the binding of different clones of humanized antibodies with CD276 (B7-H3) over-expressing cell strain CD276-CHO-K1 cells.
Figure 2 shows the binding of the B7-H3 antibody of the present invention with two human B7-H3 antigen subclasses and a murine B7-H3 antigen. mB7-H3 Ab antibody refers to the anti-human B7-H3 murine CD276 hybridoma antibody of the present invention, αhB7-H3 Ab refers to the humanized CD276 antibody of the present invention, and the hB7-H3 Ab antibody refers to a commercial B7-H3 antibody (Biolegend, cat: 351006).
Figure 3A shows the CAR positive rate of αhB7-H3 CAR-Jurkat T cells detected by flow cytometry.
Figure 3B shows the B7-H3 expression on cell surface of three gastric cancer cell strains HGC-27, AGS and KATO-III. Results show that HGC-27 and AGS are B7-H3 high-expressing cell strain, and KATO-III cell is a B7-H3 low-expressing cell strain.
Figure 3C shows the expression of CD69, CD25 and PD1 after incubation of B7-H3 CAR-Jurkat T cells with target cells.
Figure 3D shows the expression of cytokines (IL2, granzyme B) in the supernatant after incubation of B7-H3 CAR-Jurkat T cells with target cells.
Figure 4A shows a schematic structural diagram of a second-generation CAR vector constructed by using a murine B7-H3 antibody sequence targeting human B7-H3 and a humanized B7-H3 antibody sequence.
Figure 4B shows a schematic structural diagram of a lentiviral vector for CAR expression.
Figure 4C shows transfection efficiency of humanized B7-H3-CAR-T cells (αhB7-H3 CAR-T, (βhB7-H3 CAR-T) and murine B7-H3-CAR-T cells (mB 7-H3-CAR-T).
Figure 5A shows the B7-H3 antigen expression on cell surface of two pancreatic cancer cell lines, SW1990 and PANC-1.
Figure 5B shows the specific inhibition of the three B7-H3-CAR-T cells on pancreatic cancer cell lines SW1990 and PANC-1. The effect-target ratios are 1: 1 and 4: 1, respectively
Figure 5C shows the release of cytokines (IL-2, Granzyme-B and IFN-gamma) after incubation of the three B7-H3-CAR-T cells with pancreatic cancer cell lines SW 1990 and PANC-1.
Figure 6 shows *in vitro* killing of αhB7-H3 CAR-T cells on Raji cells overexpressing murine B7-H3 antigen (mB7-H3-Raji cells). Raji is a B7-H3 negative target cell.
Figure 7A shows *in vitro* killing of αhB7-H3 CAR-T on gastric cancer cell strain HGC-27 (B7-H3 positive cell strain) and KATOIII (B7-H3 negative cell strain) cells.
Figure 7B shows the release of cytokines after incubation of αhB7-H3 CAR-T with gastric cancer cell lines HGC-27 and KATOIII cells.
Figure 8A shows an experiment flowchart of treating a NSG mouse model with HGC-27 cell subcutaneous transplanted tumor by using αhB7-H3 CAR-T cells.
Figures 8B and 8C show *in vivo* inhibition of αhB7-H3 CAR-T cells on HGC-27 cells.

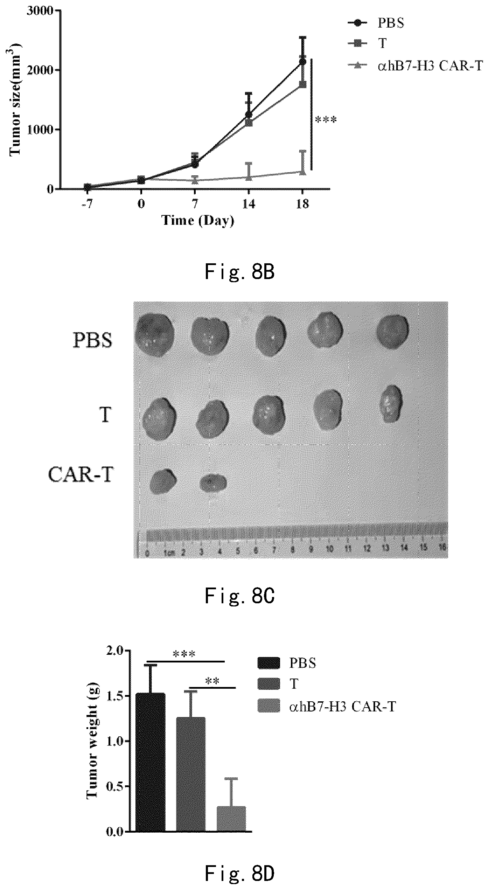

Figures 8B and 8C show *in vivo* inhibition of αhB7-H3 CAR-T cells on HGC-27 cells.
Figure 8D shows the weight change of HGC-27 tumor-bearing mice treated with αhB7-H3 CAR-T cells compared to the control group.

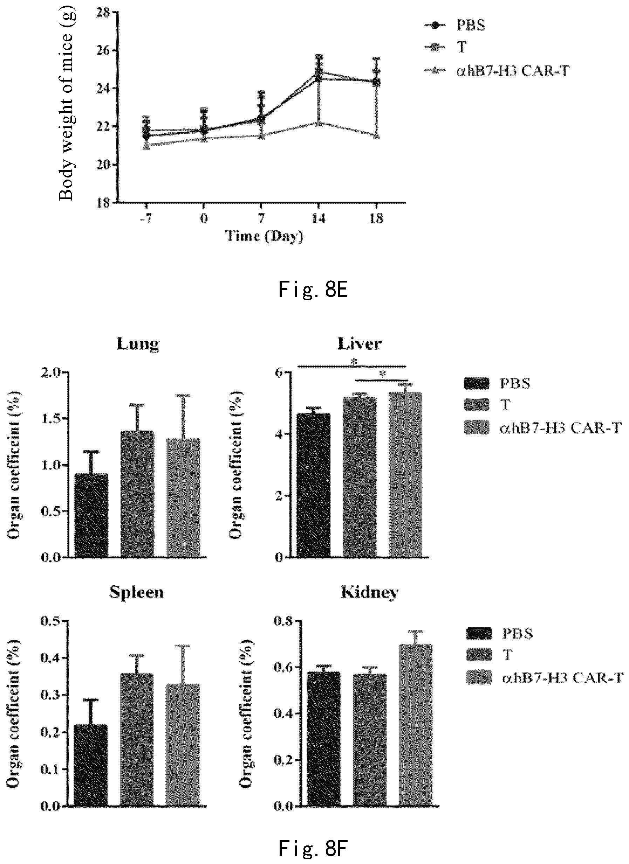

Figure 8E shows the body weight change of mice in the αhB7-H3 CAR-T group compared to the control group.
Figure 8F shows the organ coefficient change of mice in the alpha hB7-H3 CAR-T group compared to the control group.

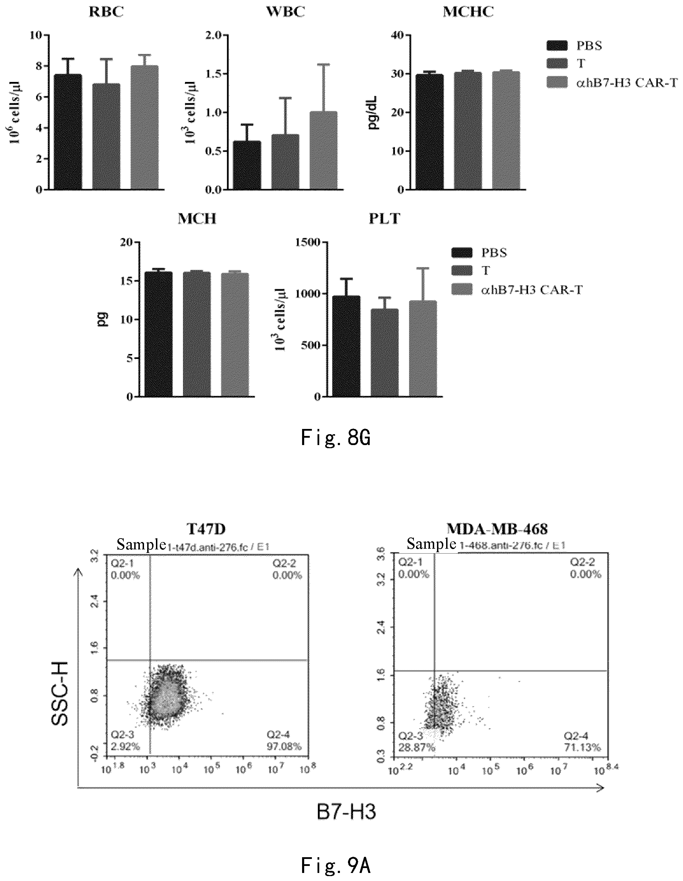

Figure 8G shows the routine blood parameter change of mice in the αhB7-H3 CAR-T group compared to the control group.
Figure 9A shows the expression of B7-H3 on cell surface of the breast cancer cell strains T47D and MDA-MB-468.

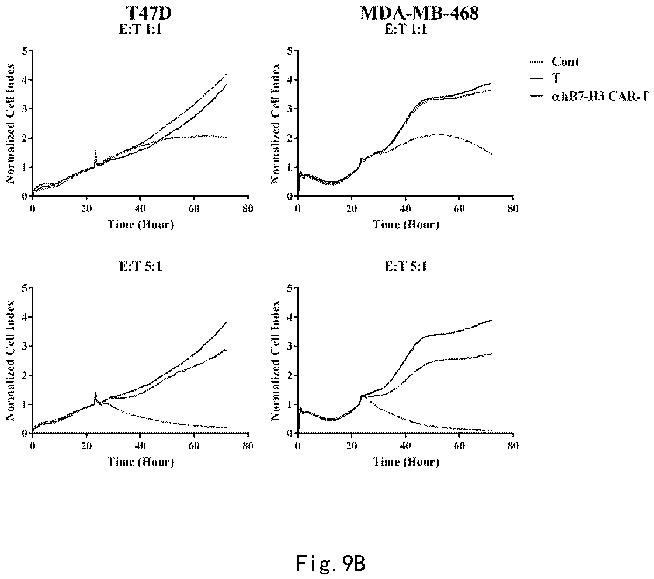

Figure 9B shows *in vitro* inhibition of αhB7-H3 CAR-T cells on breast cancer cell strains (T47D and MDA-MB-468).

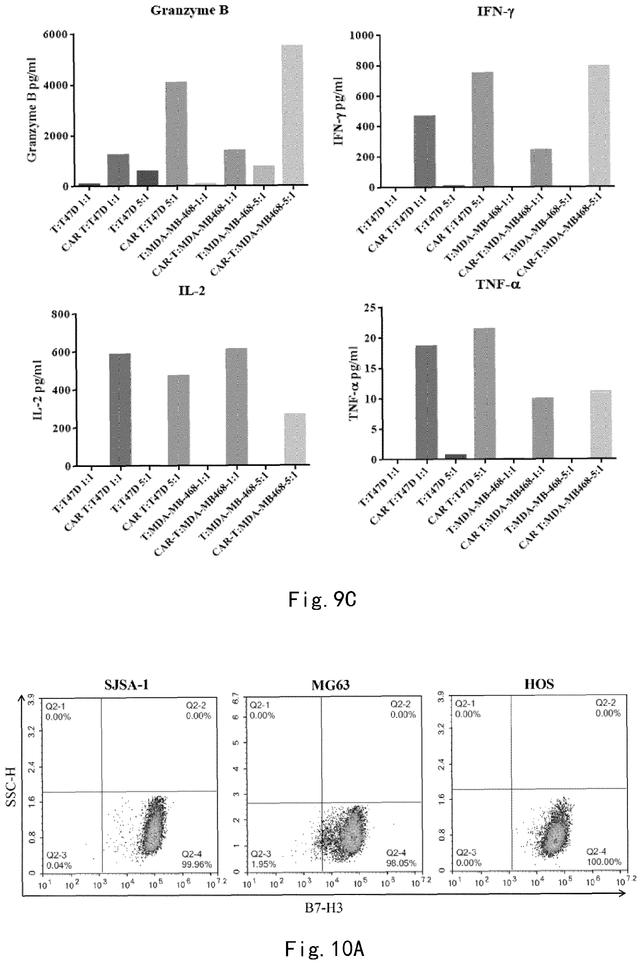

Figure 9C shows the release of cytokines (IL-2, Granzyme-B, IFN-gamma, TNF-alpha) after incubation of αhB7-H3 CAR-T cells with breast cancer cell strains.
Figure 10A shows the expression of B7-H3 on cell surface of three osteosarcoma cell strains (SJSA-1, MG63, HOS cells).

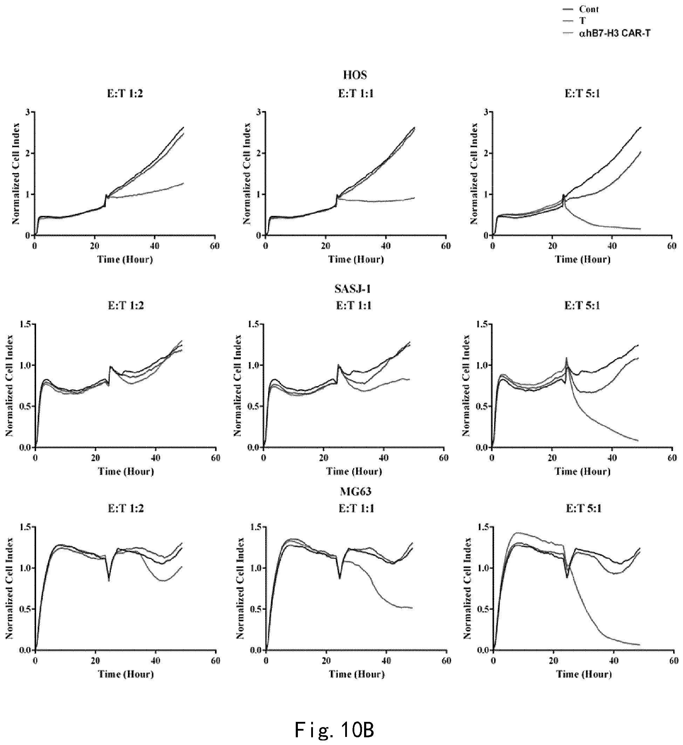

Figure 10B shows *in vitro* inhibition of alpha hB7-H3 CAR-T cells on three osteosarcoma cell strains (SJSA-1, MG63, HOS cells).

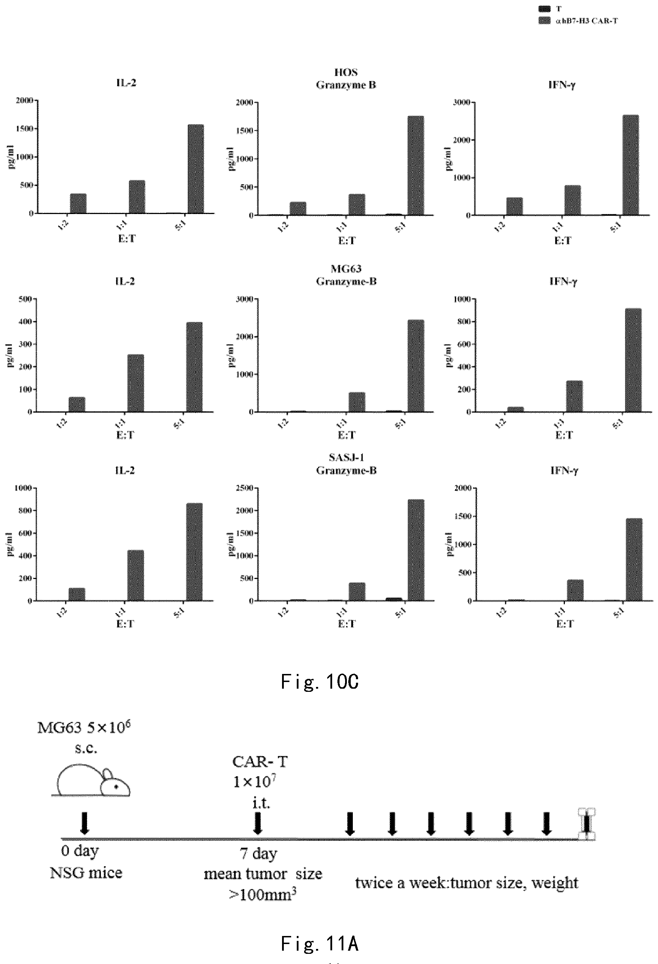

Figure 10C shows the release of the cytokines (IL-2, Granzyme-B, IFN-gamma) after incubation of the αhB7-H3 CAR-T cells with osteosarcoma cell strains.
Figure 11A shows an experiment flowchart of treating NSG mouse models subcutaneous transplanted with MG-63 cell tumor by using αhB7-H3 CAR-T cells.

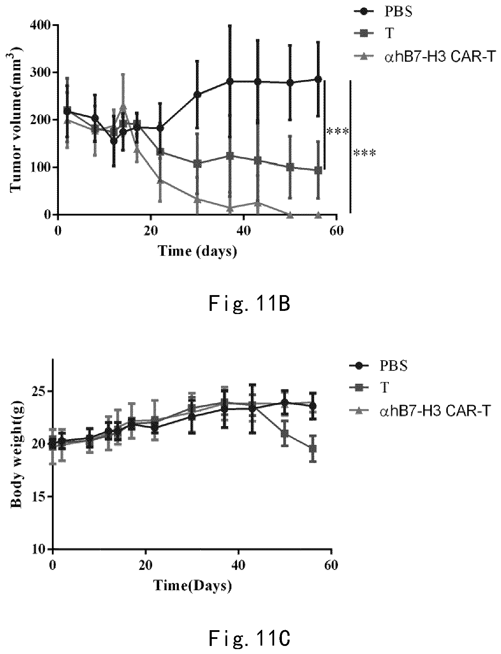

Figure 11B shows the significant inhibition of αhB7-H3 CAR-T cells on the growth and proliferation of tumor cells in mice compared to the control group.
Figure 11C shows the body weight change of mice in the αhB7-H3 CAR-T cell group compared to the control group.

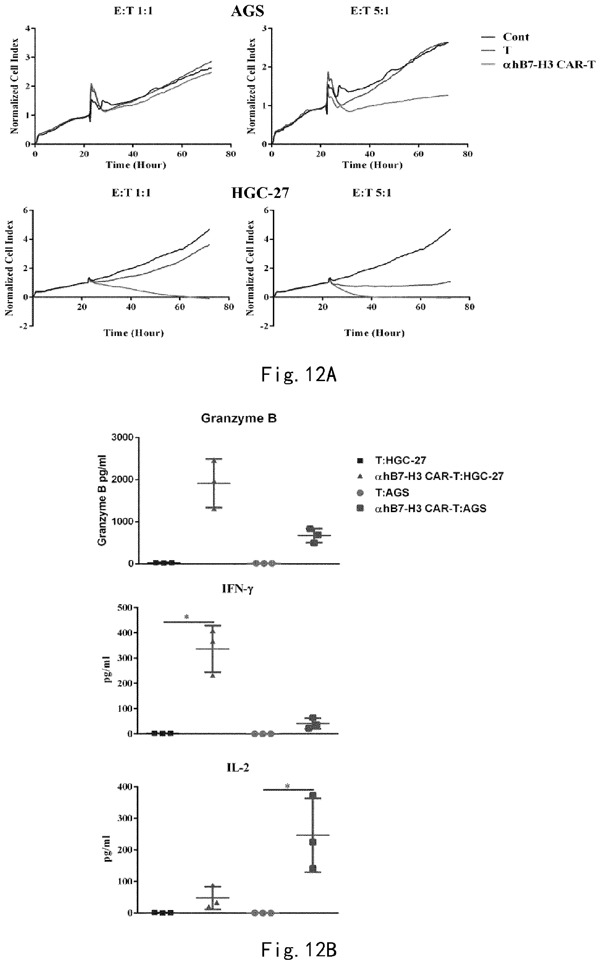

Figure 12A shows the inhibition of αhB7-H3 CAR-T cells on AGS and HGC27 cells analyzed by RTCA methods.
Figure 12B shows the release of cell factors after incubation of αhB7-H3 CAR-T cells with AGS and HGC27 cells.

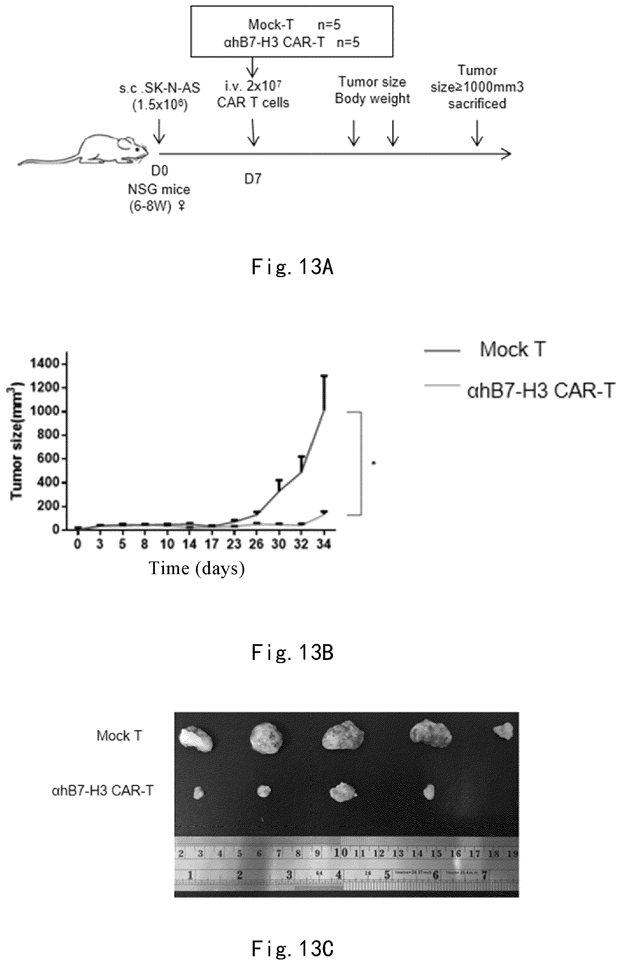

Figure 13A shows the experiment flowchart of treating NSG mouse models subcutaneously transplanted with neuroblastoma SK-N-AS cell strain by using αhB7-H3 CAR-T cells.
Figure 13B shows the change in tumor size in tumor-bearing mice treated with αhB7-H3 CAR-T cells compared to those in the control group (T cell group).
Figure 13C shows the tumor size comparison of mice in the CAR-T group and in the control group after the mice were dissected.

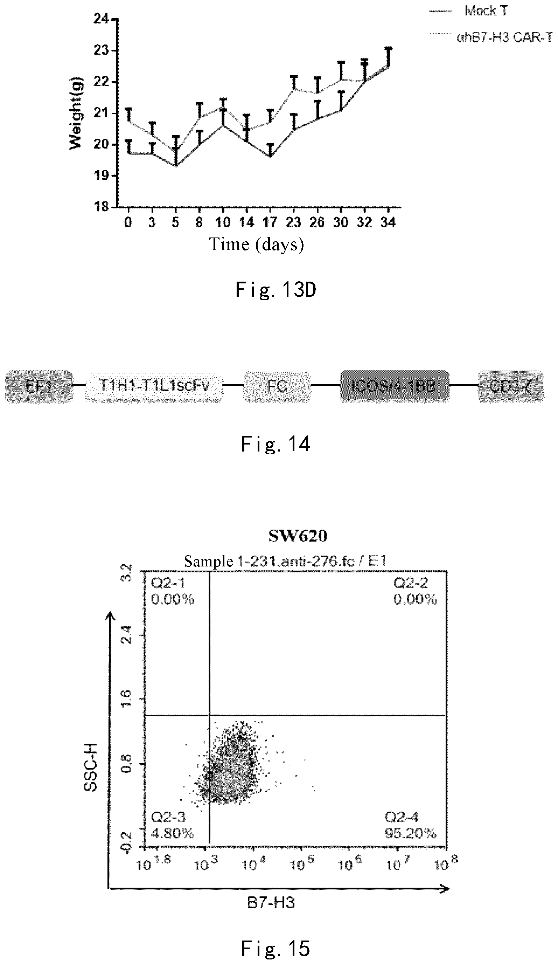

Figure 13D shows the body weight change of mice in the control group (T cell group) and αhB7-H3 CAR-T cell group.
Figure 14 shows the vector structure profile of a third-generation B7-H3-CAR.
Figure 15 shows the expression of B7-H3 on the surface of colon cancer cell strain SW620.

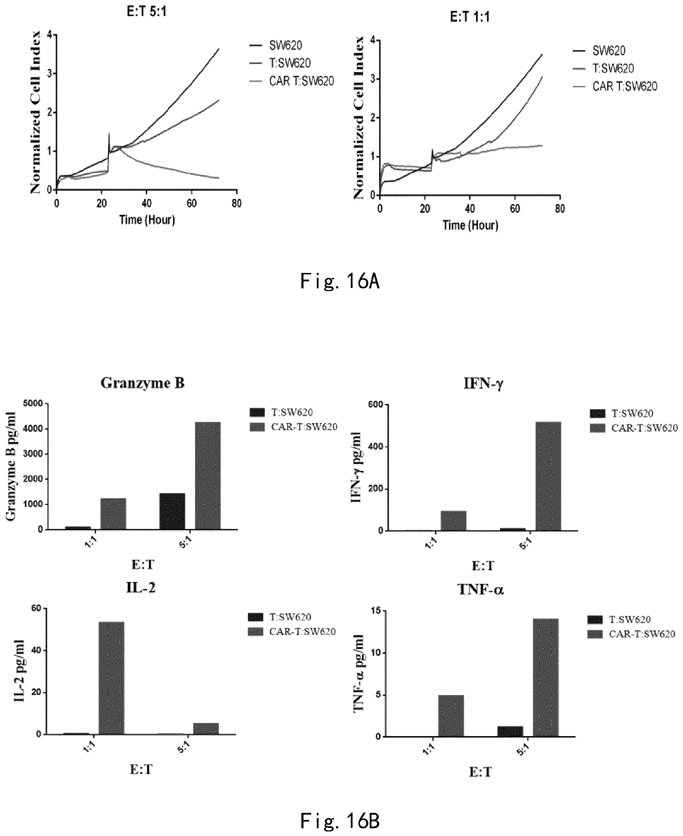

Figure 16A shows the *in vitro* inhibition of the third-generation B7-H3-CAR-T cells on SW620.
Figure 16B shows the release of cytokines after incubation of the third-generation B7-H3-CAR-T cells with SW620.

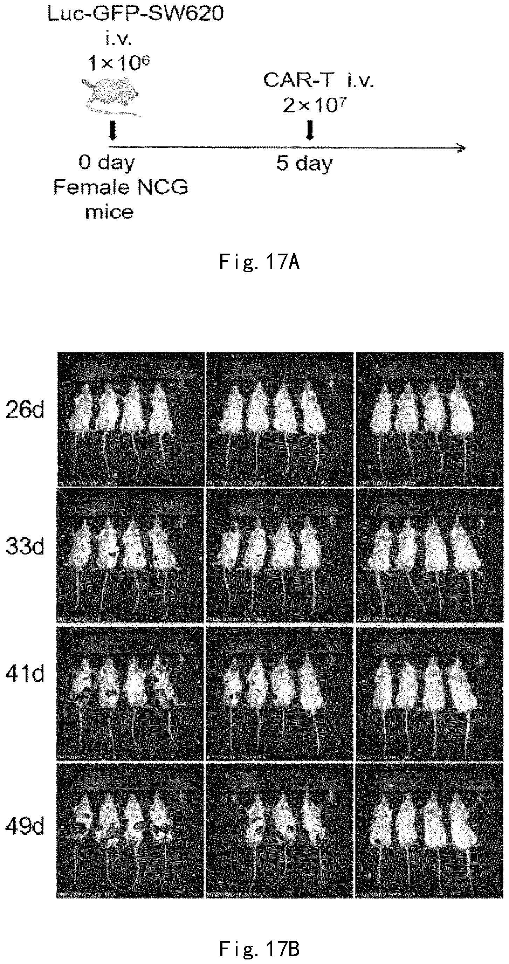

Figure 17A shows the experiment flowchart of treating SW620 tumor-bearing NCG mice with the third-generation B7-H3-CAR-T cells.
Figure 17B shows living body imaging results of mice in experimental group and control group.

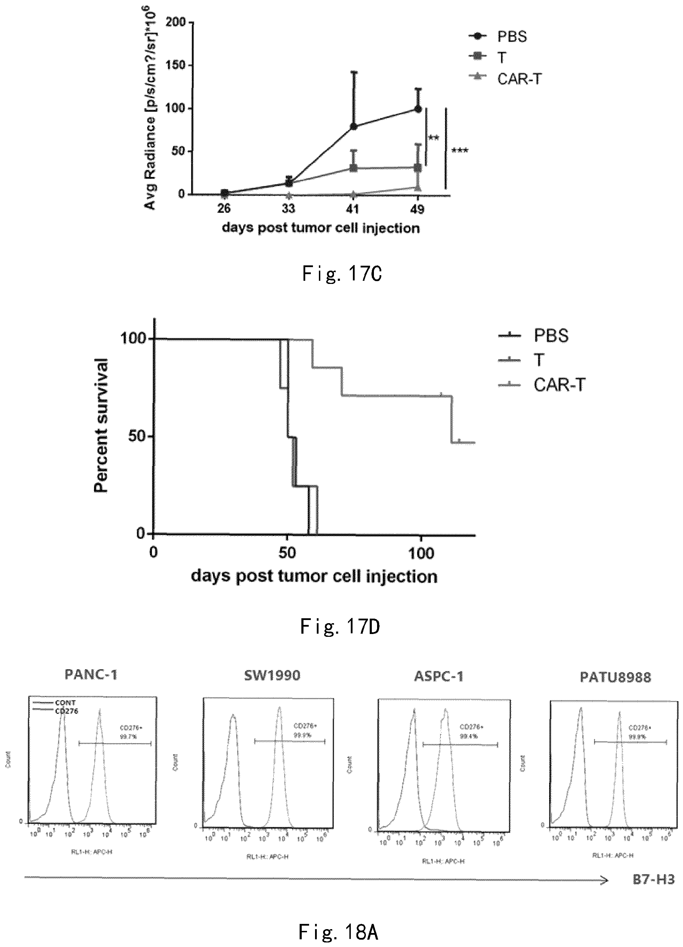

Figure 17C shows changes in tumor fluorescence signal values of mice in experimental group and control group.
Figure 17D shows the survival curves of mice in experimental group and control group.
Figure 18A shows the expression of B7-H3 of four pancreatic cancer cell strains.

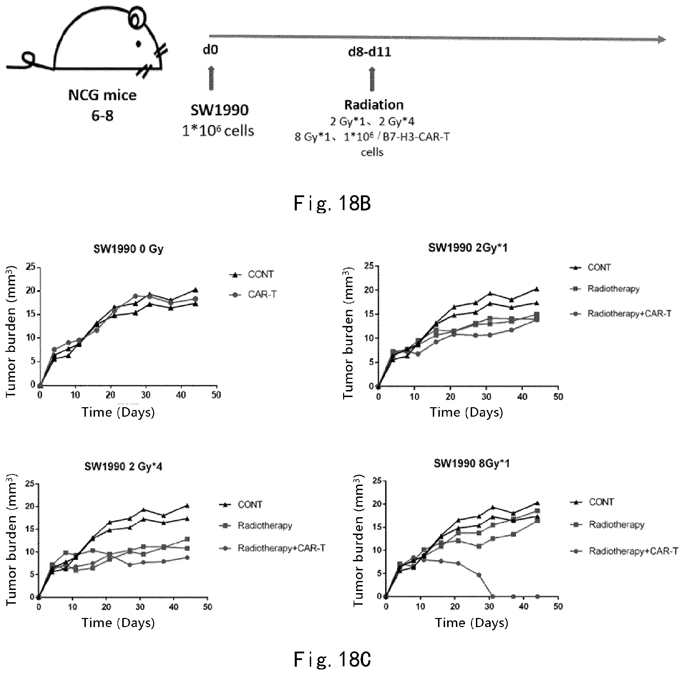

Figure 18B shows the experiment flowchart of local X-ray irradiation on tumor sites of experimental mice.
Figure 18C shows the change in tumor size of experimental mice.

### DETAILED DESCRIPTION OF EMBODIMENTS

Through extensive and intensive research, the inventors accidentally find a B7-H3 chimeric antigen receptor-modified T cell and use thereof for the first time. Specifically, the present invention provides an antibody targeting B7-H3, and a chimeric antigen receptor and an engineered immune cell prepared by using the antibody. The present invention also provides a preparation method and use of the antibody and the immune cell. The engineered immune cell of the present invention can effectively control the growth of tumor cells such as gastric cancer, osteosarcoma, triple negative breast cancer and the like, does not show distinct toxic and side effects in *in vivo* experiments, has very good safety, and shows promise to be an effective immunotherapy for treating various solid tumors. On this basis, the present invention is accomplished.

Specifically, the mB7-H3 antibody is successfully obtained by multiple rounds of immunization in BALB/c mice with h2IgB7-H3-CHO-K1 recombinant cells, fusion of hybridoma cells, and twice monoclonal screening. Then it is humanized through CDR grafting method, and two humanized B7-H3 chimeric antibodies having equivalent activity to the murine antibody, αhB7-H3 and βhB7-H3, are obtained. Corresponding second-generation CAR-T cells are respectively constructed by using single chain variable regions of the obtained mouse anti-human mB7-H3, humanized αhB7-H3 and βhB7-H3 antibodies. It is proved in the *in vitro* cytotoxicity experiment that αhB7-H3-CAR-T cells have a strong growth inhibition effect and a stronger cytokine secretion capacity on tumor cells, so that the anti-tumor activity of αhB7-H3-CAR-T cells is further studied. The anti-tumor effects of αhB7-H3 CAR-T cells on gastric cancer, osteosarcoma and triple negative breast cancer are researched in present invention. Both *in vivo* and *in vitro* experimental results show that αhB7-H3-CAR-T cell can effectively control the growth of tumor cells such as gastric cancer, osteosarcoma, triple negative breast cancer and the like, which fully indicates the efficacy of αhB7-H3-CAR-T cells in treating these solid tumors. Meanwhile, the human 2Ig-B7-H3 molecule and the murine 2Ig-B7-H3 molecule share high homology and the antibody binding experiment shows that both antigens can be recognized by αhB7-H3 antibody. However, no distinct toxic or side effect is shown in the treatment process of mice in αhB7-H3 CAR-T cell therapy group, which strongly illustrates the safety of αhB7-H3 CAR-T cell in treating solid tumors. In summary, αhB7-H3-CAR-T cell shows promise to become effective immunotherapy for treating various solid tumors.

In recent years, CAR-T therapy has made breakthrough progress in the field of tumor immunotherapy. The CAR-T therapy targeting CD19 has even more remarkable achievement for the treatment of B lymphocyte tumors, and brings good news for many patients with relapsed and refractory B lymphocytic leukemia. However, CAR-T cell immunotherapy does not achieve satisfactory effects in the field of treating solid tumors. This is primarily because of many characteristics of the solid tumor itself hindering the effect of CAR-T therapy, compared to hematological tumors. Firstly, the solid tumors have highly heterogeneous antigen expression and lack an ideal therapeutic target. Secondly, CAR-T cells are difficult to effectively home to the tumor sites. Moreover, the tumor microenvironment of solid tumor is highly inhibitory and can damage the function of CAR-T cells.

In the present invention, an mB7-H3 murine monoclonal antibody is successfully obtained by multiple rounds of immunization in BALB/c mice with h2IgB7-H3-CHO-K1 cells, hybridoma cell screening, multiple rounds of hybridoma cell clonal screening and hybridoma cell sequencing. The mB7-H3 antibody is then subjected to humanized modification, wherein the amino acid sequences of the heavy chain and light chain of the antibody are designed by using a CDR grafting scheme. Further, two humanized B7-H3 antibodies having similar activity, αhB7-H3 antibody and βhB7-H3 antibody, are screened from the obtained humanized antibodies. Next, the binding ability of αhB7-H3 antibody to the tumor cells expressing human h2IgB7H3 and h4IgB7h3 and murine m2IgB7H3 molecules on cell surface is further studied. The results show that αhB7-H3 antibody can simultaneously recognize and bind to the two human B7-H3 molecular subtypes and murine B7-H3 molecules. In addition, αhB7-H3 CAR-T cells also exhibit specific cytotoxic effects on m2IgB7H3-Raji cells. The above experimental results provide an important experimental basis for assessing the safety of CAR-T cells in mouse models.

In order to verify whether αhB7-H3 CAR is able to mediate antigen-dependent specific activation of effector cells, Jurkat T cells highly expressing αhB7-H3 CAR (alpha hB7-H3 CAR-Jurkat T) are constructed using lentivirus transducing method. Due to the fact that the Jurkat T cell is a T lymphocyte leukemia immortalized cell line and has good characteristics, it is simpler and more reliable to confirm whether the CAR can mediate cell-specific activation by constructing the CAR-Jurkat T cells. Co-incubation of αhB7-H3 CAR-Jurkat T and gastric cancer cells shows that co-incubation with B7-H3 antigen-positive tumor cells can significantly up-regulate the expression of activation molecules CD25 and CD69 and inhibitory signal molecule PD-1 of CAR-Jurkat T cells, but those co-incubated with B7-H3 antigen-negative KATO III cells only have limited expression of these molecules. This phenomenon is most likely due to the high activation degree of αhB7-H3 CAR-Jurkat T cells. The detection of cytokine content also shows similar results, while the cytokine secretion amount of αhB7-H3 CAR-Jurkat T cells is positively correlated with the antigen expression of tumor cells co-incubated therewith. These results illustrate that the αhB7-H3 CAR molecule constructed by the present invention can be specifically activated by an antigen-dependent manner.

In this invention, mB7-H3 CAR-T cells, αhB7-H3 CAR-T cells and βhB7-H3 CAR-T cells are respectively constructed by using the obtained B7-H3 antibodies, and the mB7-H3 CAR-T cells and the αhB7-H3 CAR-T cells are found to have better anti-tumor activity through *in vitro* cytotoxicity experiments. Therefore, in the following anti-tumor activity research, the *in vitro* anti-tumor activity of αhB7-H3 CAR-T cells on gastric cancer, breast cancer, osteosarcoma and other solid tumors is studied. Experiment results show that αhB7-H3 CAR-T cells can specifically lyse various B7-H3 antigen-positive tumor cells such as gastric cancer, breast cancer, osteosarcoma and the like *in vitro,* and have no distinct cytotoxicity effect on B7-H3 antigen-negative tumor cells. At the same time, it is also found that the cytotoxicity effect of αhB7-H3 CAR-T cell on tumor cells has a positive correlation with the expression quantity of tumor cell antigens. The higher the tumor cell antigen density is, the stronger the ability to stimulate the αhB7-H3 CAR-T cells to secrete cytokines. This result also illustrates that the antigen density can affect the cytotoxicity of αhB7-H3 CAR-T cells, which verified the hypothesis of Majzner et al. again that CAR-T cells have antigen-dependent treatment windows.

In the anti-tumor activity evaluation of αhB7-H3 CAR-T cells, NSG mice are used to construct a variety of allograft tumor models: gastric cancer cell subcutaneous tumor model, breast cancer cell metastasis tumor model, and osteosarcoma cell subcutaneous tumor model, for evaluating the *in vivo* anti-tumor activity of αhB7-H3 CAR-T cells and the safety of the treatment using the same. The result shows that αhB7-H3 CAR-T cells can effectively inhibit the growth of tumor cells in various xenografted tumor models, and at the same time, they do not cause distinct toxic and side effects on mice, and the weight, organ coefficient, and blood routine test in mice in the treatment group show no significant difference compared with the normal. The results of the antigen binding experiment show that the αhB7-H3 antibody can simultaneously recognize two B7-H3 molecules subtypes of human source and the B7-H3 molecule of mouse source, but the αhB7-H3 CAR-T cell does not cause distinct toxic and side effects on mice, which illustrating that the αhB7-H3 CAR-T cell does not cause cytotoxicity effect on normal mouse tissues and cells expressing B7-H3 antigen, the reason of which may be that the B7-H3 antigen amount expressed by the normal tissue is insufficient to trigger αhB7-H3 CAR-T cells to generate specific cytotoxicity. The above experimental results provide an important safety reference for the human clinical application of αhB7-H3 CAR-T cells in the future.

In conclusion, the B7-H3 monoclonal antibody with good activity is successfully obtained through mouse immunization and screening, and two humanized B7-H3 monoclonal antibodies are obtained through humanized modification. Three second-generation CAR-T cells are constructed using single chain variable regions derived from a murine antibody and two humanized antibodies, and the anti-tumor activity and safety of the αhB7-H3 CAR-T cell with good activity are deeply studied. The results show that αhB7-H3 CAR-T cells have good anti-tumor activity on gastric cancer, breast cancer and osteosarcoma *in vivo* and *in vitro.* At the same time, αhB7-H3 CAR-T cells do not cause serious toxic and side effects on mice, and these results show that αhB7-H3 CAR-T cells can treat solid tumors with good and reliable safety. The above results show that the CAR-T immunotherapy targeting B7-H3 molecules has great application prospects in the field of treatment of various solid tumors, and is expected to bring good news to vast solid tumor patients.

### Antibody

As used herein, the term "antibody" or "immunoglobulin" refers to a heterotetrameric glycoprotein having the same structural feature of about 150,000 daltons consisting of two identical light chains (L) and two identical heavy chains (H). Each light chain is linked to a heavy chain by a covalent disulfide bond, and the numbers of disulfide bonds between the heavy chains of different immunoglobulin isoforms are different. Each heavy and light chain also has regularly spaced intrachain disulfide bonds. One end of each heavy chain has a variable region (VH) followed by a plurality of constant regions. There is a variable region (VL) at one end of each light chain and a constant region at the other end. The constant region of the light chain corresponds to the first constant region of the heavy chain, and the variable region of the light chain corresponds to the variable region of the heavy chain. An interface is formed between the variable regions of the light and heavy chains by particular amino acid residues.

As used herein, the term "variable" means that some certain portions of the variable region of an antibody differ in sequence and contribute to the binding and specificity of each particular antibody to its particular antigen. However, the variability is not evenly distributed throughout the antibody variable region. It is concentrated in three regions in the light and heavy chain variable regions called complementarity determining regions (CDRs) or hypervariable regions. The more conserved portions of the variable regions are referred as framework regions (FRs). The variable regions of the natural heavy and light chains each comprises four FR regions, which are in a substantially β-folded configuration, and are linked by three CDRs that form the linker ring, and, in some cases, form a partial β-folded structure. The CDRs in each chain stand close together through FR regions and form the antigen-binding site of the antibody together with the CDRs of the other chain (see Kabat et al., NIH Publ. No. 91-3242, Vol. I, 647-669 (1991)). Constant regions are not directly involved in the binding of the antibodies to the antigens, but they exhibit different effector functions, such as antibody-dependent cellular cytotoxicity involved in antibodies.

The "light chain" of vertebrate antibodies (immunoglobulins) can be classified into one of two distinct categories (called kappa and lambda) based on the amino acid sequence of their constant region. Immunoglobulins can be classified into different types based on the amino acid sequence of their heavy chain constant region. There are mainly 5 types of immunoglobulins: IgA, IgD, IgE, IgG, and IgM, some of which can be further divided into subclasses (isotypes), such as IgG1, IgG2, IgG3, IgG4, IgA, and IgA2. The heavy chain constant regions corresponding to different types of immunoglobulins are called α, δ, ε, γ, and µ, respectively. The subunit structures and three-dimensional configurations of different types of immunoglobulins are well-known to those in this field.

In general, the antigen-binding properties of an antibody can be described by three specific regions located in the heavy chain variable region, referring as variable regions (CDRs), and separated into four framework regions (FRs). The sequences of four FRs amino acids are relatively conservative and do not directly participate in the binding reaction. A cyclic structure are formed by these CDRs which are close to each other in the spatial structure by the β-folds formed by the FRs between them, and the CDRs on the heavy chains and the CDRs on the corresponding light chains constitute the antigen-binding sites of the antibody. The amino acid sequence of the same type of antibody can be used to determine which amino acids have constituted the FR or CDR region.

The present invention includes not only intact antibodies, but also immunologically active fragments of antibody fragments or fusion proteins formed by antibodies and other sequences. Therefore, the present invention also includes fragments, derivatives and analogs of the antibodies.

In the present invention, antibodies include murine, chimeric, humanized, or fully human antibodies prepared by techniques well known to those skilled in the art. Recombinant antibodies, such as chimeric and humanized monoclonal antibodies, including human and non-human parts, can be prepared using DNA recombinant techniques well known in the art. They are all useful antibodies. A chimeric antibody is a molecule in which different parts come from different animal species, such as a chimeric antibody comprising a variable region of a monoclonal antibody from mice and a constant region from a human immunoglobulin (see, e.g., U.S. Patents 4816567 and 4816397, which are entirely incorporated herein by reference). Humanized antibodies refer to antibody molecules derived from non-human species which have one or more complementarity determining regions (CDRs) derived from non-human species and framework regions derived from human immunoglobulin molecules (see U.S. Patent 5585089, which is entirely incorporated herein by reference). These chimeric and humanized monoclonal antibodies can be prepared using well-known DNA recombination techniques in the field.

In the present invention, the antibody may be monospecific, bispecific, trispecific, or more multispecific.

In the present invention, the antibody of the present invention also includes conservative variants thereof, which means that compared with the amino acid sequence of the antibody of the present invention, there are at most 10, preferably at most 8, more preferably at most 5, most preferably at most 3 amino acids replaced by amino acids with the same or similar properties to form a polypeptide. These conservatively variant polypeptides are preferably produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-B7-H3 antibody

The present invention provides an antibody targeting B7-H3 with high specificity and high affinity, which comprises a heavy chain and a light chain, wherein the heavy chain comprises a heavy chain variable region (VH) amino acid sequence, and the light chain comprises a light chain variable region (VL) amino acid sequence.

In another preferred embodiment, the antibody comprises a heavy chain variable region and/or a light chain variable region.
wherein the heavy chain variable region comprises the following complementarity determining regions (CDRs):
a CDR1 shown in SEQ ID NO: 2;
a CDR2 shown in SEQ ID NO: 3; and
a CDR3 shown in SEQ ID NO: 4;
the light chain variable region comprises the following complementarity determining regions (CDRs):
   a CDR1' shown in SEQ ID NO: 6;
   a CDR2' shown in SEQ ID NO: 7; and
   a CDR3' shown in SEQ ID NO: 8.

In another preferred embodiment, any one of the above amino acid sequences further includes a derivative sequence which is optionally added, deleted, modified, and/or substituted with at least one amino acid, and can retain the binding affinity to B7-H3.

In another preferred embodiment, the sequence formed by adding, deleting, modifying, and/or substituting at least one amino acid has preferably at least 80% amino acid sequence homology, preferably at least 85%, preferably at least 90%, and preferably at least 95%. More preferably, the number of added, deleted, modified, and/or substituted amino acids can be 1-7, more preferably 1-5, more preferably 1-3, and more preferably 1-2.

The antibody of the present invention may be a double stranded or a single stranded antibody, and can be selected from an animal derived antibody, a chimeric antibody, and a humanized antibody, more preferably a humanized antibody, a human-animal chimeric antibody, and more preferably a fully humanized antibody.

The antibody derivative described in the present invention can be a single chain antibody, and/or an antibody fragment, such as Fab, Fab', (Fab')₂ or other known antibody derivatives in the field, as well as any one or more of IgA, IgD, IgE, IgG, and IgM antibodies or other subtypes of antibodies.

Among them, the animal is preferably a mammal, such as a mouse.

The antibody of the present invention may be a chimeric antibody, a humanized antibody, a CDR grafted and/or modified antibody, targeting human B7-H3.

### Preparation of the antibody

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, DNA sequences encoding the protein of the invention (or fragments thereof, or derivatives thereof) can be completely obtained by chemical synthesis. The DNA sequence can then be introduced into a variety of existing DNA molecules (or vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell may be a prokaryotic cell, such as a bacterial cell; or a lower eukaryotic cell, such as a yeast cell; or a higher eukaryotic cell, such as an animal cell. Preferred animal cells comprise, but are not limited to, CHO-S, HEK-293 cells.

In general, under conditions suitable for expression of the antibody according to the present invention, the transformed host cell is cultured. Then, the antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an *in vitro* binding assay (such as radioimmunoassay (RIA) or enzyme-linked immunosorbent assay (ELISA)). The binding affinity of a monoclonal antibody, for example, can be determined by using Scatchard analysis described in Munson et al., Anal. Biochem, 107:220 (1980).

The antibody of the present invention can be expressed intracellularly or on the cell membrane, or be secreted out of the cell. If desired, recombinant proteins can be isolated and purified by various separation methods using their physical, chemical and other properties. These methods are well known to those skilled in the art. Examples of such methods include, but are not limited to, conventional renaturation treatments, treatment with a protein precipitant (salting-out method), centrifugation, osmosis cell disruption, super-treatment, ultracentrifugation, molecular sieve chromatography (gel filtration), adsorption chromatography, ion-exchange chromatography, high performance liquid chromatography (HPLC) and various other liquid chromatography techniques and combinations of these methods.

### Antibody-Drug Conjugates (ADCs)

The present invention also provides an antibody-drug conjugate (ADC) based on the antibody of the present invention.

Typically, the antibody-drug conjugate comprises the antibody and an effector molecule, wherein the antibody is conjugated, preferably chemically conjugated to the effector molecule. Preferably, the effector molecule is a therapeutically active drug. In addition, the effector molecule may be one or more of a toxic protein, a chemotherapeutic drug, a small-molecule drug or a radionuclide.

The antibody of present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that forms a covalent bond between the effector molecule and the antibody, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g., a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker may be a degradable or non-degradable linker. Typically, the degradable linker is easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g., lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g., linkers that are hydrolyzed at a pH of below 5.5, such as hydrazone linkers) and linkers that are degraded under reducing conditions (e.g., disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g., iodo-, bromo-, or chloro-substituted) amide; a halogenated (e.g., iodo-, bromo- or chloro-substituted) ester; a halogenated (e.g., iodo-, bromo-, or chloro-substituted) methyl ketone, a benzyl halide (e.g., iodide, bromide or chloride); vinyl sulfone, pyridyl disulfide; a mercury derivative such as 3,6-di-(mercurymethyl)dioxane, wherein the counter ion is an acetate, a chloride, or a nitrate; and polymethylene dimethyl sulfide thiosulfonate. The linker may include, for example, a maleimide linked to an antibody via thiosuccimide.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA groove binding agents, DNA alkylation agents and tubulin inhibitors, and typical cytotoxic drugs include, for example, auristatins, camptothecins, duocarmycins, etoposides, maytansines and maytansinoids (e.g., DM1 and DM4), taxanes, benzodiazepines, or benzodiazepine-containing drugs (e.g., pyrrolo[1,4]benzodiazepines (PBDs), indolinobenzodiazepines, and oxazolidinobenzodiazepines) and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, as described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), connecting an antibody to a drug-linker compound.

In certain embodiments, the method according to the present invention comprises: under conditions sufficient to form an antibody-linker conjugate, connecting an antibody to a bifunctional linker compound. In these embodiments, the method according to the present invention further comprises: under conditions that the drug moiety is sufficient to be covalently linked to the antibody via a linker, connecting the antibody-linker conjugate to the drug moiety.

In some embodiments, an antibody-drug conjugate (ADC) has a formula as follows: 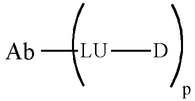 wherein,
Ab is an antibody,
LU is a linker;
D is a drug;
and the subscript p is a value selected from 1 to 8.

### Chimeric antigen receptor

The chimeric antigen receptor (CAR) of the present invention comprises an extracellular domain, a transmembrane domain, and an intracellular domain. The extracellular domain comprises a target-specific binding element (also known as an antigen binding domain). The intracellular domain includes a co-stimulatory signaling region and a ζ chain. The co-stimulatory signaling region refers to a part of the intracellular domain that includes a co-stimulatory molecule. The co-stimulatory molecule is a cell surface molecule required for efficient response of lymphocytes to antigens, rather than an antigen receptor or its ligand.

A linker (or linking peptide) can be incorporated between the extracellular domain and the transmembrane domain of the CAR, or between the cytoplasmic domain and the transmembrane domain of the CAR. As used herein, the term "linker" generally refers to any oligopeptide or polypeptide that plays a role of linking the transmembrane domain to the extracellular domain or the cytoplasmic domain in a polypeptide chain. The linker may comprise 0-300 amino acids, preferably 2-100 amino acids and most preferably 3-50 amino acids.

In a preferred embodiment of the present invention, the extracellular domain of the CAR provided by the present invention comprises an antigen binding domain targeting B7-H3. When the CAR of the present invention is expressed in T cell, antigen recognition can be performed based on antigen binding specificity. When the CAR binds to its associated antigen, it affects tumor cell, causing tumor cell to fail to grow, to death or to be affected otherwise, causing the patient's tumor burden to shrink or eliminate. The antigen binding domain is preferably fused to one or more intracellular domains of the co-stimulatory molecule and the ζ chain. Preferably, the antigen binding domain is fused with an intracellular domain of a combination of a 4-1BB signaling domain and a CD3ζ signaling domain.

As used herein, both "antigen binding domain" and "single chain antibody fragment" refer to a Fab fragment, a Fab' fragment, a F(ab')₂ fragment, or a single Fv fragment having antigen binding activity. An Fv antibody contains a heavy chain variable region and a light chain variable region of the antibody, but does not have a constant region, and has the smallest antibody fragment with all antigen binding sites. Generally, an Fv antibody also contains a peptide linker between VH and VL domains and can form a structure required for antigen binding. The antigen binding domain is usually a scFv (single chain variable fragment). The size of a scFv is generally 1/6 of that of a complete antibody. A single chain antibody is preferably an amino acid chain sequence encoded by a single nucleotide chain. As a preferred method of the present invention, the scFv specifically recognizes B7-H3.

As for the hinge region and the transmembrane region (transmembrane domain), the CAR can be designed to comprise a transmembrane domain fused to the extracellular domain of the CAR. In one embodiment, a transmembrane domain that is naturally associated with one of the domains in the CAR is used. In some embodiments, a transmembrane domain may be selected or modified by amino acid substitutions to avoid binding such domain to the transmembrane domain of the same or different surface membrane proteins, thereby minimizing the interaction with other members of the receptor complexes.

The intracellular domain in the CAR of the present invention comprises the signaling domain of 4-1BB and the signaling domain of CD3ζ.

### Vector

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically.

The present invention also provides vectors in which the expression cassette of the present invention is inserted. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells, such as hepatocytes. They also have the advantage of low immunogenicity.

In brief, the expression cassette or nucleic acid sequence of the present invention is typically and operably linked to a promoter, and incorporated into an expression vector. The vector is suitable for replication and integration in eukaryotes. A typical cloning vector contains transcription and translation terminators, an initiation sequence, and promoters useful for regulation of the expression of the desired nucleic acid sequence.

The expression construct of the present invention may also be used for nucleic acid immune and gene therapy, using standard gene delivery protocols. Methods for gene delivery are known in the art, see, e.g., U.S, Pat. Nos. 5,399,346, 5,580,859, and 5,589,466, which are incorporated by reference herein in entirety. In another embodiment, the present invention provides a gene therapy vector.

The nucleic acid can be cloned into various vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al, (2001, Molecular Cloning: A Laboratory Manual, Cold Spring Harbor Laboratory, New York), and in other virology and molecular biology manuals. Viruses which are useful as vectors include but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326, 193).

A number of viral based systems have been developed for transferring a gene into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentiviral vectors are used.

Additional promoter elements, e.g., enhancers, can regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the present invention should not be limited to the use of constitutive promoters. Inducible promoters are also contemplated as part of the present invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionein promoter, a glucocorticoid promoter, a progesterone promoter, and a tetracycline promoter.

In order to assess the expression of a CAR polypeptide or portions thereof, the expression vector to be introduced into a cell may also contain either a selectable marker gene or a reporter gene or both of two to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5 flanking regions showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors may be derived from lentivirus, poxviruses, herpes simplex virus I, adenoviruses and adeno-associated viruses, and the like. See, for example, U.S. Pat, Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*)*.* In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

In a preferred embodiment of the present invention, the vector is a lentiviral vector.

### Therapeutic application of CAR-T

The present invention comprises therapeutic applications by using cells (e.g., T cells) transduced with a lentiviral vector (LV) encoding the expression cassette of the present invention. The transduced T cells can target the tumor cell marker B7-H3, synergistically activate T cells, and cause T cell immune responses, thereby significantly increasing the killing efficiency against tumor cells.

Therefore, the present invention also provides a method for stimulating a T cell-mediated immune response to a target cell population or tissue in a mammal comprising a step of administering to the mammal a CAR-T cell of the present invention.

In one embodiment, the present invention comprises a cell therapy, wherein T cells from autologous patient (or heterologous donor) are isolated, activated and genetically modified to generate CAR-T cells, and then injected into the same patient. The probability of graft-versus-host-disease in the way is extremely low, and antigens are recognized by T cells in a non-MHC-restricted manner. In addition, one CAR-T can treat all cancers that express the antigen. Unlike antibody therapy, CAR-T cells are able to replicate *in vivo* and result in long-term persistence that can lead to sustained tumor control.

In one embodiment, the CAR-T cells of the present invention can undergo robust *in vivo* T cell expansion and can persist for an extended period. In addition, the CAR mediated immune response may be part of an adoptive immunotherapy approach in which CAR-modified T cells induce an immune response specific to the antigen binding moiety in the CAR. For example, an anti-B7-H3 CAR-T cell elicits an immune response specific against cells expressing B7-H3.

Although the data disclosed herein specifically disclose lentiviral vector comprising anti-B7-H3 scFv, hinge and transmembrane domains, and 4-1BB and CD3ζ signaling domains, it is contemplated that the present invention include any number of variations for each of the components of the construct as described elsewhere herein.

Cancers that may be treated include tumors that have not been vascularized or have not yet been vascularized, and tumors that have been vascularized. Cancers may include non-solid tumors (such as hematological tumors, for example, leukemia and lymphomas) or solid tumors. Types of cancers to be treated with the CAR of the present invention include, but are not limited to, cancer, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemia, including acute leukemia (such as acute lymphocytic leukemia, acute myelocytic leukemia, acute myelogenous leukemia and myeloblasts, promyeiocytic, myelomonocytic, monocytic and erythroleukemia), chronic leukemia (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors (such as sarcomas and carcinomas) include fibrosarcoma, myxosarcoma, liposarcoma, mesothelioma, malignant lymphoma, pancreatic cancer and ovarian cancer.

The CAR-modified T cells of the present invention may also serve as a type of vaccine for *ex vivo* immunization and/or *in vivo* therapy in a mammal. Preferably, the mammal is a human.

With respect to *ex vivo* immunization, at least one of the following occurs *in vitro* prior to administering the cells into a mammal: i) expanding the cells, ii) introducing a nucleic acid encoding a CAR to the cells, and/or iii) cryopreservation of the cells.

*Ex vivo* procedures are well known in the art and are discussed more fully below. Briefly, cells are isolated from a mammal (preferably a human) and genetically modified (i.e., transduced or transfected *in vitro*) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of *ex vivo* immunization, the present invention also provides compositions and methods for *in vivo* immunization to elicit an immune response directed against an antigen in a patient.

The present invention provides a method for treating tumors comprising administering to a subject in need thereof, a therapeutically effective amount of the CAR-modified T cells of the present invention.

The CAR-modified T cells of the present invention may be administered either alone, or as a pharmaceutical composition in combination with diluents and/or with other components such as IL-2, IL-17 or other cytokines or cell populations. Briefly, the pharmaceutical composition of the present invention may comprise a target cell population as described herein, in combination with one or more pharmaceutically or physiologically acceptable carriers, diluents or excipients. Such compositions may comprise buffers such as neutral buffered saline, sulfate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

The pharmaceutical composition of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount", "an anti-tumor effective amount", "an tumor-inhibiting effective amount", or "therapeutic amount" is indicated, the precise amount of the composition of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹ cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al,, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (i.v.) injection, or intraperitoneally. In one embodiment, the T cell composition of the present invention is administered to a patient by intradermal or subcutaneous injection. In another embodiment, the T cell composition of the present invention is preferably administered by i.v. injection. The compositions of T cells may be injected directly into a tumor, lymph node, or site of infection.

In certain embodiments of the present invention, cells activated and expanded using the methods described herein, or other methods known in the art where T cells are expanded to therapeutic levels, are administered to a patient in conjunction with (e.g., before, simultaneously or after) any number of relevant treatments, including but not limited to treatment with agents such as antiviral therapy, cidofovir and interleukin-2, Cytarabine (also known as ARA-C), or natalizumab treatment for MS patients, or efalizumab treatment for psoriasis patients, or other treatments for PML patients. In further embodiments, the T cells of the present invention may be used in combination with chemotherapy, radiation, immunosuppressive agents, such as cyclosporin, azathioprine, methotrexate, mycophenolate, and FK506, antibodies, or other immunotherapeutic agents. In a further embodiment, the cell composition of the present invention is administered to a patient in conjunction with (e.g., before, simultaneously or after) bone marrow transplantation, or the use of chemotherapy agents such as, fludarabine, external-beam radiation therapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or after surgery.

The dosage of the above treatments to be administered to a patient will vary with the precise nature of the condition being treated and the recipient of the treatment. The scaling of dosages for human administration may be performed according to practices in the field. In general, 1×10⁶ to 1×10¹⁰ of the modified T cells of the present invention can be applied to patients by means of, for example, intravenous infusion in each treatment or each course of treatment.

### CAR T Formulation

The present invention provides a formulation (or preparation) comprising the CAR-T cell of the present invention, and a pharmaceutically acceptable carrier, diluent or excipient. In one embodiment, the formulation is a liquid formulation. Preferably, the formulation is an injection. Preferably, the concentration of the CAR-T cells in the formulation is 1×10³-1×10⁸ cells/ml, more preferably 1×10⁴-1×10⁷ cells/ml.

In one embodiment, the formulation may comprises buffers such as neutral buffered saline, sulfate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. The formulation of the present invention is preferably formulated for intravenous administration.

### Pharmaceutical composition

The present invention further provides a composition. In preferred embodiments, the composition is a pharmaceutical composition comprising the above-mentioned antibody, or an active fragment, a fusion protein or an ADC thereof, or a corresponding CAR-T cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated. The formulated pharmaceutical composition may be administered by conventional routes, including (but not limited to): intratumoral, intraperitoneal, intravenous, or topical administration.

The antibody of the present invention can also be used for cell therapy by expressing the nucleotide sequence in the cell. For example, the antibody is used for chimeric antigen receptor T cell immunotherapy (CAR-T) and the like.

The pharmaceutical composition according to the present invention can be directly used for binding to a TF protein molecule, and thus can be used for preventing and treating diseases such as tumors. In addition, other therapeutic agents can also be used at the same time.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection or a solution, should be prepared under aseptic conditions. The administration amount of an active ingredient is a therapeutically effective amount, for example, about 1 µg per kilogram of body weight to about 5 mg per kilogram of body weight daily. In addition, the polypeptide according to the present invention may also be used in combination with an additional therapeutic agent.

When a pharmaceutical composition is used, a safe and effective amount of immune conjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, a specific amount should also depend on the factors such as administration route and physical conditions of a patient, which fall into the skills of skilled physicians.

### Use for detection and the kit

The antibody or the conjugate thereof of the present invention can be used for detection, for example, for detection of samples to provide diagnostic information.

In the present invention, the specimens (samples) used include cells, tissue samples and biopsy specimens. The term "biopsy" used in the present invention shall include all kinds of biopsy known to those skilled in the art. Therefore, the biopsy used in the present invention may include, for example, resection samples of tumors, tissue samples prepared by endoscopic methods or organ puncture or needle biopsy.

The samples used in the present invention include fixed or preserved cells or tissue samples.

The present invention also provides a kit containing the antibody (or a fragment thereof) of the present invention. In a preferred embodiment of the present invention, the kit further includes a container, instructions for use, buffer, and the like. In a preferred embodiment, the antibody of the present invention can be immobilized on a detection plate.

The present invention also provides a use of the antibody of the present invention, such as for preparing diagnostic agents or medicaments for preventing and/or treating B7-H3 positive tumors.

### The main advantages of the present invention include:

(a) the CAR-T cell of the present invention has good anti-tumor activity against gastric cancer, breast cancer, osteosarcoma, neuroblastoma, colon cancer and pancreatic cancer *in vivo* and *in vitro;*
(b) the CAR-T cell of the present invention has good and reliable safety in treating solid tumors;
(c) the CAR-T cell of the present invention is highly activated, and co-incubation of that with B7-H3 antigen-positive tumor cells can significantly upregulate the expression of activated molecules CD25 and CD69 and inhibitory signal molecule PD-1 of the CAR-T cell.
(d) The CAR-T cell of the present invention can significantly inhibit the growth and proliferation of tumor cells in mice bearing gastric cancer, osteosarcoma, neuroblastoma or colon cancer cell strain, and has significant anti-tumor effects *in vivo.*

The invention is further illustrated below in conjunction with specific examples. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

### General Materials and Methods:

### Cell Lines

K562 cells, Raji cells, h4IgB7H3-Raji cells, h2IgB7H3-K562 cells, m2IgB7H3-Raji cells, and h2IgB7-H3-CHO-K1 recombinant cells are provided by iCarTab Biotechnology (Suzhou) Co. Ltd. 293T cells, T47D cells, MDA-MB-231 cells, MDA-MB-468 cells and AGS cells are purchased from ATCC. HGC-27 cells, KATO III cells, SUN-1 cells, MG63 cells, and HOS cells are purchased from Shanghai Institute of Cell, Chinese Academy of Sciences. K562 cells, Raji cells, h4IgB7H3-Raji cells, h2IgB7H3-K562 cells, m2IgB7H3-Raji cells, h2IgB7-H3-CHO-K1 recombinant cells, HGC-27 cells, KATO III cells and SUN-1 cells are cultured in 1640 culture medium containing 10% fetal bovine serum. 293T cells, T47D cells, MDA-MB-231 cells, MDA-MB-468 cells, HOS cells, MG63 cells, and AGS cells are cultured in 1640 culture medium containing 10% fetal bovine serum. The above cells are all cultured in an incubator containing 5% CO2 at 37 °C.

### Antibody Screening

Five Balb/c mice were immunized respectively by intraperitoneal injection of 5×10⁶ h2IgCHO-K1-CD276 recombinant cells (provided by iCarTab Biotechnology (Suzhou) Co. Ltd.), repeated every 14 days. 100 µl of blood was taken from the tail vein of each mouse 10 days after the third intraperitoneal injection, for the immune titer detection of B7-H3 antibodies in the serum of each mouse by ELISA. Mice with the best immune titer were selected and booster-immunized by infusion of 5×10⁶ recombinant cell strains through the tail vein. The mouse spleens were isolated for hybridoma fusion after immunization for three days. Mice with the best immune titer were sacrificed by cervical dislocation, and their spleens were obtained in a sterile environment. B cell single cell suspension was prepared and mixed with SP2/0 myeloma cells in a ratio of 1: 1, and cell fusion was performed by using a Bio-rad Gene Pulser electroporation system. After fusion, cells were resuspended with DMEM medium containing 20% fetal bovine serum and HAT, and seeded into a 96 well plate. After 10 days of fusion, the medium was replaced with HT culture medium. After two days of culture, the supernatant was taken for detection of the specific antibody. The taken culture medium was incubated with an ELISA well plate pre-coated with the target antigen, and was identified according to a standard ELISA operation step. Wells with higher OD values were selected for the second- and third-round subcloning screening. The titer of the B7-H3 antibodies in the supernatant of screened cell in each round was detected by ELISA. 5×10⁶ final screened hybridoma cells were taken to extract total RNA. After reverse transcription of the total RNA into cDNA sample by using a reverse transcription kit, the heavy chain and light chain variable regions of the antibodies were amplified by using hybridoma sequencing primers through PCR. Then TA cloning was performed by subcloning the fragments obtained by PCR into PMD-19T vectors. After blue-white selection, 10 clones of each chain were picked respectively for sequencing. The finally obtained antibody sequences were analyzed.

### Antibody Humanization

Design of humanized antibody sequence: humanization design was performed according to the amino acid sequence information of the heavy chain and the light chain of the target antibody obtained by the above mentioned sequencing. The CDR region sequences of the original antibody remained unchanged, and different humanized antibody templates were selected for the heavy chain and light chain respectively, according to the results of the germline alignment and the results of antibody structure simulation, then reverse mutations were carried out in the humanized frame region. Five light chain and five heavy chain candidate sequences of humanized antibody were designed. After gene synthesis of the antibody heavy and light chain sequences, they were respectively subcloned into pcDNA3.1-IgG1Fc and pcDNA3.1-IgKc expression vectors. The constructed humanized light and heavy chain vectors were transfected into 293F-SVP16 cells by using LVTransm transfection method. After three days of continuous culture, the culture supernatant was collected and filtered with a filter membrane of 0.45 µm, and the filtrate was transferred to a sterile centrifuge tube for flow cytometry detection. Each humanized antibody was incubated with CHO-K1 cells and h2IgB7-H3-CHO-K1 recombinant cells at room temperature for 1 h and washed 3 times with PBS. Then 2 µl of PE-labeled Anti-Human IgG was added to the corresponding groups, mixed well, and incubated at room temperature in dark place for 30 min, and then washed 3 times with PBS. 500 µL of PBS were added to each group to resuspend cells, and the flow cytometry was then performed. The humanized antibodies with better activity were screened out according to the flow cytometry result, and the affinity of the humanized antibodies were detected. The binding capacity of the non-humanized and humanized antibodies to the target protein B7-H3 antigen was detected by fixing the B7-H3-His recombinant protein on a CM5 chip by using a 10 mM acetic acid coupling buffer and using the above prepared positive humanized antibodies identified by flow cytometry and human-murine chimeric antibodies as the mobile phase.

### Vector Construction

In the present experiment, a mouse anti-human B7-H3 antibody was successfully screened out, and two humanized antibodies which has equivalent activity thereto, αhB7-H3 and βhB7-H3, were obtained by humanization. mB7-H3-CAR, αhB7-H3 CAR and βhB7-H3 CAR structures were designed using single-chain variable regions of the mouse antibody and the two humanized antibodies, respectively, and were synthesized by means of gene synthesis. Subsequently, the synthesized mB7-H3-CAR, αhB7-H3 CAR and βhB7-H3 CAR sequences were respectively subcloned to pCAR-AT-Free lentivirus vectors, and were sequentially named as αhB7-H3 CAR lentiviral vector and βhB7-H3 CAR lentiviral vector.

### Lentivirus Preparation

The mB7-H3-CAR, the αhB7-H3 CAR lentivirus and the βhB7-H3 CAR lentivirus are prepared by using the 293T-S suspension cell PEI lentivirus packaging method, and the lentivirus stock solution was harvested 48 hours after plasmid transfection. After the stock solution was centrifuged, it was filtered by 0.22 µm. The filtered virus was concentrated through ultracentrifugation. After concentration, the supernatant was discarded, and DMEM medium containing 0.5% DNase I was added, and the virus was resuspended by vortex oscillation. The virus resuspended solution was collected, the impurities were removed by centrifugation, and the virus resuspended solution was split and cryopreserved at -80 °C for later use.

### Construction of mX7-H3-CAR, αhB7-H3 CAR-T cells and βhB7-H3 CAR-T cells

The peripheral blood used in the present invention was provided by healthy volunteers. PBMCs were extracted using Ficoll gradient centrifugation. The obtained PBMCs were resuspended using TexMACA GMP Medium containing IL -7 and IL -15 and then seeded into a 24-well plate, and 20 µl of TransAct reagent was added to each well for activation. After 48 h, the activated and amplified T cells were collected, centrifuged and resuspended and then seeded into a 48-well plate, a corresponding amount of virus was added at an MOI of 20, and the volume was adjusted to 100 µl. After 12 h, the supernatant was removed by centrifugation, and a fresh culture medium was added to continue to culture the cells. After seven days of transfection, cells were collected and washed 3 times with PBS. Cells were added with 100 µL of tEGFR antibody diluted with PBS at 1:1000 in advance (final concentration of antibody: 1 ug/mL), resuspened and incubated at room temperature for 30 min. Afer incubation, the supernatant was discarded through centrifugation, and cells were washed 3 times with PBS. Cells were resuspended by adding 500 µL of PBS, and then detected by flow cytometer.

### Construction of αhB7-H3 CAR-Jurkat T Cells

5×10⁵ Jurkat T cells were seeded to a 48-well plate, a corresponding amount of virus was added at the MOI of 20, and the culture system was adjusted to 200 µl. After 12 h, cells were collected by centrifugation, resuspended with fresh culture medium, and re-seeded into a 24-well plate for continued culture. After 5 days of transfection, cells were collected and washed three times using PBS. Cells were added with 100 µL of tEGFR antibody diluted with PBS at 1:1000 in advance, resuspened and incubated at room temperature for 30 min. Afer incubation, the supernatant was discarded through centrifugation, and cells were washed 3 times with PBS. Cells were resuspended by adding 500 µL of PBS, and then detected by flow cytometer.

### αhB7-H3 CAR-Jurkat T Cell Specific Activation Experiment

The HGC-27, AGS and KATO III cells were collected by pancreatin digestion and washed three times with PBS, then resuspended using a CFSE solution diluted with PBS at 1:1000, incubated at 37°C for 15 min. Cells were centrifuged to discard supernatant, washed 3 times with PBS, then resuspended with culture medium, and the cell density was adjusted to 4×10⁵ cells/ml. The above tumor cell suspension was seeded into a 24-well plate at 500 µl/well. The corresponding effector cells were added to the wells at an effect-target ratio of 1:1, and the final culture system was adjusted to 1 ml/well, and placed in a CO₂ incubator for co-culture for 24 h. Three replicated wells were set up in each experimental group. Cells in each well were collected after 24h, and after centrifugation, 100 µL of supernatant from each well was collected for cytokine detection. The remaining supernatant was discarded. Cells were washed three times with PBS, and then resuspended with 100 µl PBS, respectively. 1 µL of PD-1 antibody, CD69 antibody and CD25 antibody were respectively added to the corresponding tubes; after mixing well, the mixture was incubated at 37°C for 30 min. After 30 min, the supernatant was discarded through centrifugation, and cells were washed 3 times with PBS. Cells were resuspended by adding 500 µL of PBS into each tube respectively, and then detected by flow cytometer.

### Antigen Expression Detection of Tumor Cell Antigen B7-H3

After the cells to be detected were collected by centrifugation, the supernatant was discarded, and the cells were washed 3 times with PBS. The cells were resuspended with 100 µL of PBS solution, added with 1 µL of αhB7-H3 antibody, and incubated at 37°C for 30 min. After 30 min, the supernatant was discarded through centrifugation, and cells were washed 3 times with PBS and resuspended by adding 100 µL of IgG1-Fc antibody diluted with PBS at 1:1000 in advance, and incubated at 37°C for 30 min. A blank control tube, a secondary antibody control tube and a detection tube were set. After 30 min, the supernatant was discarded through centrifugation, and cells were washed 3 times with PBS. Cells were resuspended by adding 500 µL of PBS, and then detected by flow cytometer.

### In Vitro Cytotoxicity Experiment

CFSE 7-AAD flow cytometry: Tumor target cells were collected, washed three times with PBS, labeled using a pre-diluted CFSE (5,6-carboxyfluorescein diacetate succinimidy ester) solution, and incubated at 37°C for 15 min. Cells were collected by centrifugation, the supernatant was discarded, and the cells were washed 3 times with PBS. The culture medium was added to resuspend cells, and the cell density was adjusted to 2×10⁵ cells/ml, then the cells were seeded into a 24-well plate. The corresponding effector cells were added according to the set effect-target ratio, mixed well, and then placed in a CO₂ incubator for culture for 24 h. Three replicated wells were set up in each experimental group. The cells were collected after 24h, centrifuged to discard the supernatant, washed 3 times with PBS, and then resuspended with 500 µl PBS. 1 µL of 7-AAD (7-amino actinomycin D) was added to the corresponding tube, and the cells were detected and analysis by a flow cytometer. CFSE and 7-AAD double positive cells represent dead tumor cells, and the killing efficiency of the corresponding effector cells can be calculated accordingly.

RTCA method: Target cells were collected by centrifugation, and adjusted to a cell density of 2×10⁵/mL with culture medium. Culture medium was added to the E-Plate at 50 µm/well for baseline equilibrium. After equilibrium, 50 µL of target cell suspension was added to the E-plate plate, standing for 30 min in a biosafety cabinet. After 30 min, the plate was put into an RTCA instrument for data collection once every 5 minutes. After 24 hours, effector cells were added according to the E:T ratio, and cell culture system in each well was adjusted to 200 µl, then the plate was placed back to the RTCA instrument for continued monitoring of data collection once every 15 min.

### Cytokine Detection

The cell co-culture supernatant in each cytotoxicity experiment was collected, and the secretion of interferon γ (IFN-γ), granzyme B, tumor necrosis factor α (TNF-α) and IL-2 of each effector cell were detected and analyzed by CBA detection. Human granzyme β-CBA Flex Set D7 kit, human IFN-γ CBA Flex Set E7 kit, human IL-2 cba Flex Set A4 and human TNF-CBA Flex Set D9 for cytokine detection were purchased from BD Company.

### Animal Model

In the present invention, NOD-Prkdcs-cidIl2rgtm1/Bcgen (B-NSG) mouse models xenografted with a variety of tumors were constructed for the assessment of the effectiveness and safety of αhB7-H3 CAR-T cell therapy. All experimental animals used in the study were female B-NSG mice of 6-8 weeks old.

Establishment of HGC-27 gastric cancer xenografted NSG mouse tumor model: 1×10⁶ HGC-27 cells were inoculated subcutaneously on the right back of the mouse by subcutaneous injection. Mice body weight and tumor sizes were measured twice a week. When the mice tumor reached an average volume of 100 mm³, the mice were randomly divided into 3 groups: PBS group, T cell group, and αhB7-H3 CAR-T cell group, with 5 mice in each group. Then, 1×10⁷ corresponding effector cells were infused to mice in the T cell group and αB7-H3 CAR-T cell group by intratumoral injection, and mice in the PBS group were injected with PBS of an equal volume to the effector cell suspension at the same time. When the tumor size of any mouse exceeded 2000 mm³, mice in each group were sacrificed and dissected to observe the changes of organ coefficients and tumor size. In addition, before the sacrifice of mice, blood was collected from the orbital veins of each group of mice for five-class blood routine analysis.

Establishment of MG63 osteosarcoma xenografted NSG mouse tumor model: 5×10⁶ MG63 cells were mixed with matrigel and inoculated subcutaneously on the right back of the mouse by subcutaneous injection. Mice body weights were measured twice a week. When the mice tumor reached an average volume of 100 mm³, the mice were randomly divided into 3 groups: PBS group, T cell group, and αhB7-H3 CAR-T cell group, with 5 mice in each group. Then, 1×10⁷ corresponding effector cells were infused to mice in the T cell group and αB7-H3 CAR-T cell group by intratumoral injection, and mice in the PBS group were injected with PBS of an equal volume to the effector cell suspension at the same time. Weight change and survival were continuously observed. After 108 days of treatment, each group of mice were sacrificed and dissected to count the number and size of tumors in mice.

### Data Analysis

Data obtained in the present invention were statistically analyzed by using GraphPad Prism 5 software. The Student's Test was applied for analysis, with a P value less than 0.05 being considered to have a statistical difference. *, **, *** and **** represent P<0.05, <0.01, <0.001 and <0.0001, respectively, to show the degree of difference between the two sets of data.

### Example 1 B7-H3 Monoclonal Antibody Screening and Humanized Modification

B7-H3-CHO-K1 recombinant cells were used to immunize 5 Balb/c mice once every 14 days. The B7-H3 antibody titer in serum of each mouse was detected 10 days after the third immunization. Mice with the best antibody titer were screened out, and spleen cells thereof were taken and fused with hybridoma cells. Subsequently, a mouse anti-human mB7-H3 antibody having a strong binding ability to the antigen was successfully obtained through two-rounds of clone screening, and the antibody sequences thereof were obtained by sequencing the hybridoma cells.

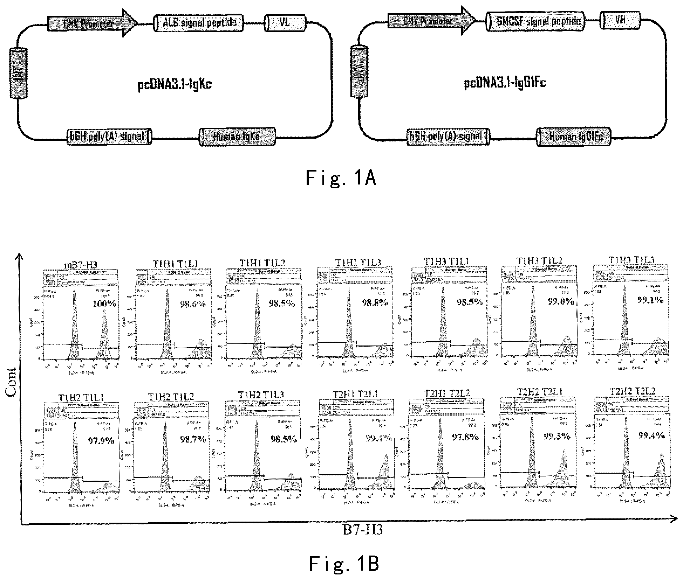

The humanization design was carried out according to the amino acid sequences of the heavy chain and the light chain of the mB7-H3 antibody by using CDR grafting method. The designed humanized candidate antibody was subjected to gene synthesis. The humanized heavy chain variable region was subcloned to the pcDNA3.1-IgG1Fc expression vector, and the humanized light chain variable region was subcloned to the pcDNA3.1-IgKc expression vector (Fig. 1A). The constructed humanized light chain and heavy chain expression vectors are respectively combined in pairs and transiently co-transfected into 293F cells (cultured without serum). After 72 hours of transfection, the expressed supernatant was collected for FACS detection.

As a result, the humanized antibody constructed in this example are able to bind the recombinant 2hIgB7-H3-CHO-K1 cells (Fig. 1B), and the binding ability is equivalent to that of the chimeric antibody. Two humanized antibodies T1H1-T1L1 and T2H1-T2L1 were selected for affinity detection and the results are shown in Table 1. T1H1-T1L1 and T2H1-T2L1 antibody were named as αhB7-H3 and (βhB7-H3 antibody, respectively.

**Table 1**

| | Ka (M-1s-1) | Kd (s-1) | KD (M) |
|---|---|---|---|
| mB7-H3 | 3.643× 10⁴ | 0.002581 | 7.083 × 10⁻⁸ |
| B7-H3 T1H1-T1L1 | 2.482 × 10⁴ | 0.002823 | 1.138 × 10⁻⁷ |
| B7-H3 T2H1-T2L1 | 3.966 ×10⁴ | 0.002887 | 7.279 × 10⁻⁸ |

The sequence numbers of the main antibodies involved in the present example are shown in Table 2.

**Table 2**

| Antibody No. | Heavy Chain | | | | Light Chain | | | |
|---|---|---|---|---|---|---|---|---|
| | Variable Region | CDR1 | CDR2 | CDR3 | Variable Region | CDR1' | CDR2' | CDR3' |
| mB7-H3 | 1 | 2 | 3 | 4 | 5 | 6 | 7 | 8 |
| T1H1-T1L1 | 9 | 2 | 3 | 4 | 10 | 6 | 7 | 8 |
| T2H1-T2L1 | 11 | 2 | 3 | 4 | 12 | 6 | 7 | 8 |

### Example 2 Recognition of Human B7-H3 Antigen and Mouse B7-H3 Antigen by αhB7-H3 Antibody

After the αhB7-H3 antibody was obtained, whether the αhB7-H3 antibody could bind to various antigens was studied by using h4IgB7H3-Raji cells, h2IgB7H3-K562 cells and m2IgB7H3-Raji cells expressing two human B7-H3 subtypes and a mouse B7-H3 subtype, respectively.

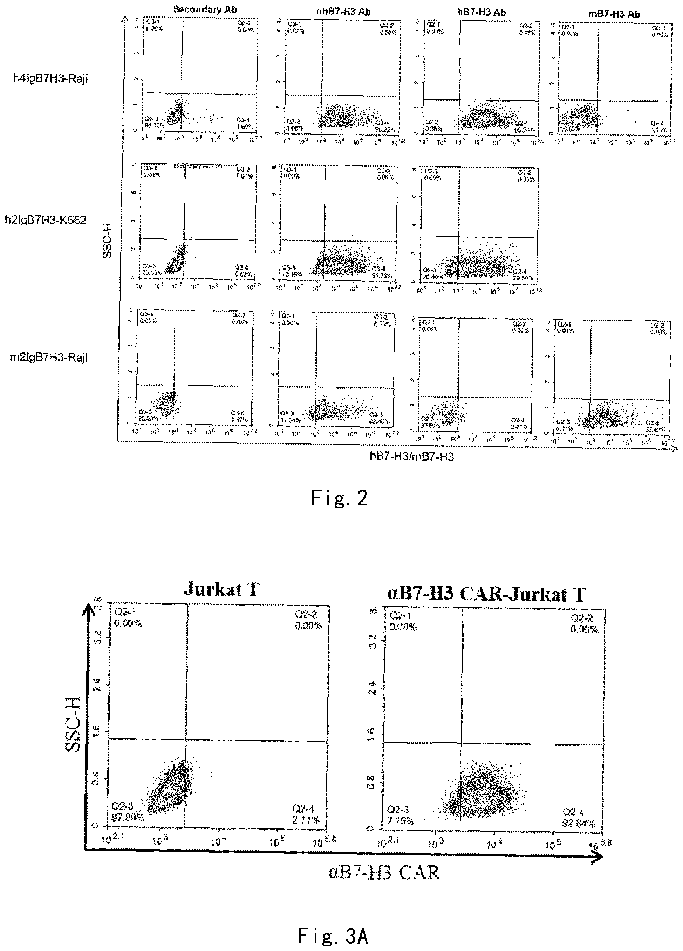

The result is shown in Fig. 2. The commercialized B7-H3 antibody (Biolegend, cat: 351006) can only bind to the corresponding B7-H3 subtype, while the B7-H3 antibody of the present invention can recognize both two human B7-H3 antigen subtypes and the mouse B7-H3 antigen. This result shows that the B7-H3 of the present invention has a wider antigen recognition capability, and can provide great convenience for verifying the safety in mouse models.

### Example 3 αHb7-H3 CAR-Jurkat T Cells Can Be Specifically Activated By B7-H3 Antigen-Positive Tumor Cells

In order to further study the specificity of the αhB7-H3 CAR molecule,αhB7-H3 CAR-Jurkat T cells were constructed through lentivirus transfection, and the positive rate of αhB7-H3 CAR-Jurkat T cells was detected as 92.84% by flow cytometry (Fig. 3A).

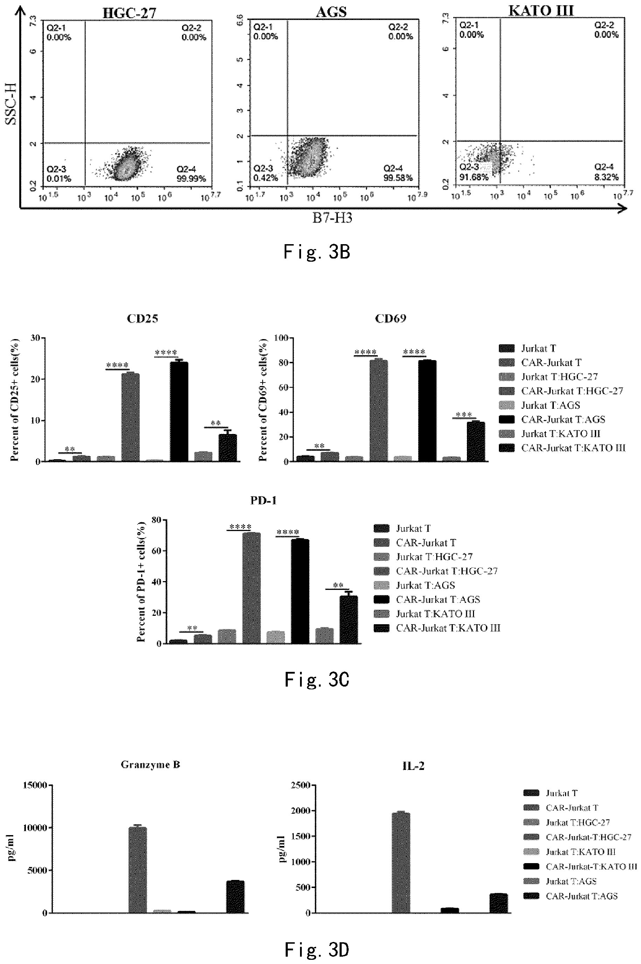

Three strains of gastric cancer cells, HGC-27, AGS and KATO-III, were used in this experiment to verify the specificity of αhB7-H3 CAR-Jurkat T cells, and their B7-H3 antigen positive rates are shown in Fig. 3B.

Jurkat T and αhB7-H3-CAR-Jurkat T cells were incubated with CFSE-labeled HGC-27, AGS and KATO-III cells at an effect-target ratio of 1:1 for 24 hours, respectively. Then each set of Jurkat T cells and αhB7-H3-CAR-Jurkat cells were collected, and the expression of CD69, CD25 and PD-1 on cell surfaces were detected.

The results show that after co-incubation with HGC-27 cells and AGS cells, the expression of CD69 and CD25 and PD-1 molecules are significantly up-regulated compared with Jurkat cells, and after co-incubation with KATO III cells, the expression of CD69, CD25 and PD-1 on Jurkat T and B7-H3 CAR-Jurkat T cells are not significantly up-regulated (Fig. 3C). In addition, the detection result of the cytokine content in the supernatant shows that after co-incubation with HGC-27 cells, the secretion of IL-2 and granzyme B by αhB7-H3 CAR-Jurkat T cells are significantly enhanced, and the KOTA III co-incubation group does not exhibit such phenomenon (Fig. 3D). These results indicate that the activation of αhB7-H3-CAR-Jurkat T cells highly depends on the antigen.

### Example 4 Construction of B7-H3 CAR-T Cells

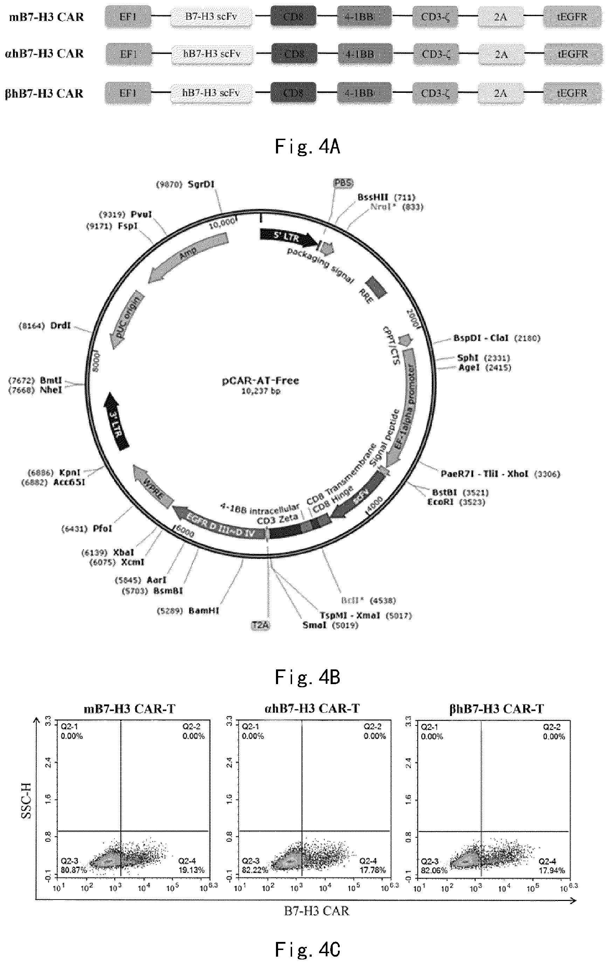

Second-generation mB7-H3 CAR, αhB7-H3 CAR and βhB7-H3 CAR were constructed using murine mB7-H3 antibodies, and humanized αhB7-H3 and βhB7-H3 antibodies respectively, the structures of which are shown in Fig. 4A, wherein the tEGFR protein can be used for the detection of CAR molecular expression rate. They were then subcloned to lentiviral vectors (Fig. 4B). Then mB7-H3 CAR-T cells, αhB7-H3 CAR-T cells and βhB7-H3 CAR T cells were respectively constructed through lentivirus transfection. Each of B7-H3 CAR-T cells were dyed using a mouse anti-human tEGFR antibody on the tenth day of transfection, and the positive rates thereof were detected by a flow cytometer.

The results show that the positive rates of mB7-H3 CAR-T cells, αhB7-H3 CAR-T cells and βhB7-H3 CAR T cells are 19.13%, 17.78% and 17.94%, respectively (Fig. 4C).

### Example 5 Cytotoxicity Comparison Between αhB7-H3 CAR-T Cells and βhB7-H3 CAR-T Cells

The growth inhibitory effect of αhB7-H3 CAR-T cells and βhB7-H3 CAR-T cells on pancreatic cancer SW1990 cells and PANC-1 cells were compared using RTCA method.

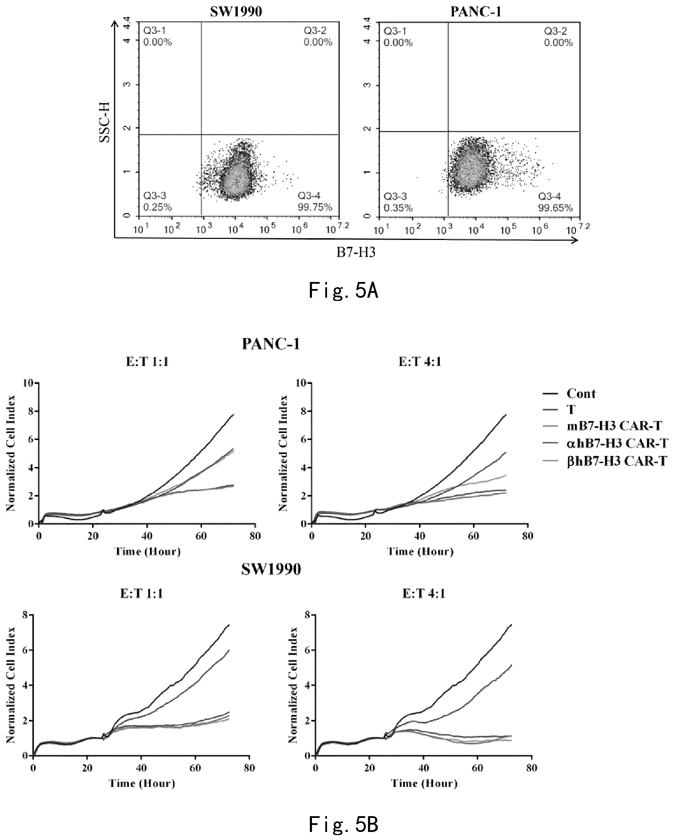

The B7-H3 antigen expression of the two pancreatic cancers are shown in Fig. 5A. The results show that compared with T cells, mB7-H3 CAR-T cells, αhB7-H3 CAR-T cells and βhB7-H3 CAR-T cells have specific inhibitory effects on SW1990 cells and PANC-1 cells, and meanwhile, mB7-H3 CAR-T cells and αhB7-H3 CAR-T cells have a stronger inhibitory effect (Fig. 5B).

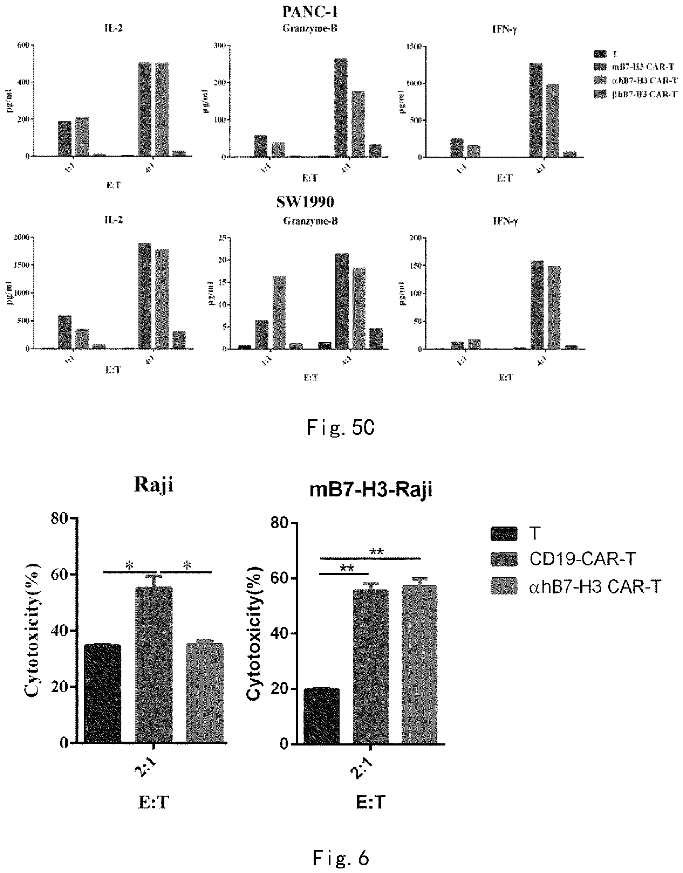

In the experiment, the detection results of cytokine content in the supernatant also show that mB7-H3 CAR-T cells and αhB7-H3 CAR-T cells have a stronger cytokine secretion capacity than βhB7-H3 CAR-T cells (Fig. 5C). The above results show that αhB7-H3 CAR-T cells have better anti-tumor activity than βhB7-H3 CAR-T cells. Therefore, αhB7-H3 CAR-T cells were selected for further research in subsequent experiments.

### Example 6 AB7-H3 CAR-T Cells Have Specific Cytotoxic Effects on M2igb7h3-Raji Cells

The antibody binding experiment results show that the αhB7-H3 antibody can recognize the human and murine B7-H3 antigens, and thus further verifies the cytotoxicity of αB7-H3 CAR-T cells to Raji cells and m2IgB7H3-Raji cells. Raji cells naturally express CD19 antigen, and thus CD19 CAR-T cells were selected as positive control cells.

The results show that the αhB7-H3 CAR-T cells show significant specific cytotoxicity only to m2IgB7H3-Raji cells, while CD19-CAR T cells exhibit specific cytotoxicity to both Raji cells and m2IgB7H3-Raji cells (Fig. 6). The above experimental results show that the αhB7-H3 CAR-T cells can specifically recognize and kill tumor cells expressing murine B7-H3 antigen, and provide an important basis for subsequent *in vivo* experiments for evaluating the safety of αhB7-H3 CAR-T cells in mice.

### Example 7 αB7-H3 CAR-T Cells for Treatment of Gastric Cancer

B7-H3 molecules are highly expressed in tumor tissues of gastric cancer patients, and the expression of B7-H3 molecules is closely related to metastasis of gastric cancer cells and poor prognosis of patients. Therefore, the anti-tumor activity of αhB7-H3 CAR-T cells to gastric cancer cells was evaluated.

HGC-27 and KATO-III cells were co-incubated with T cells and αhB7-H3 CAR-T cells respectively for 24 hours in *in vitro* experiment, and the target ratios were set as 1:1, 5: 1 and 10: 1, respectively.

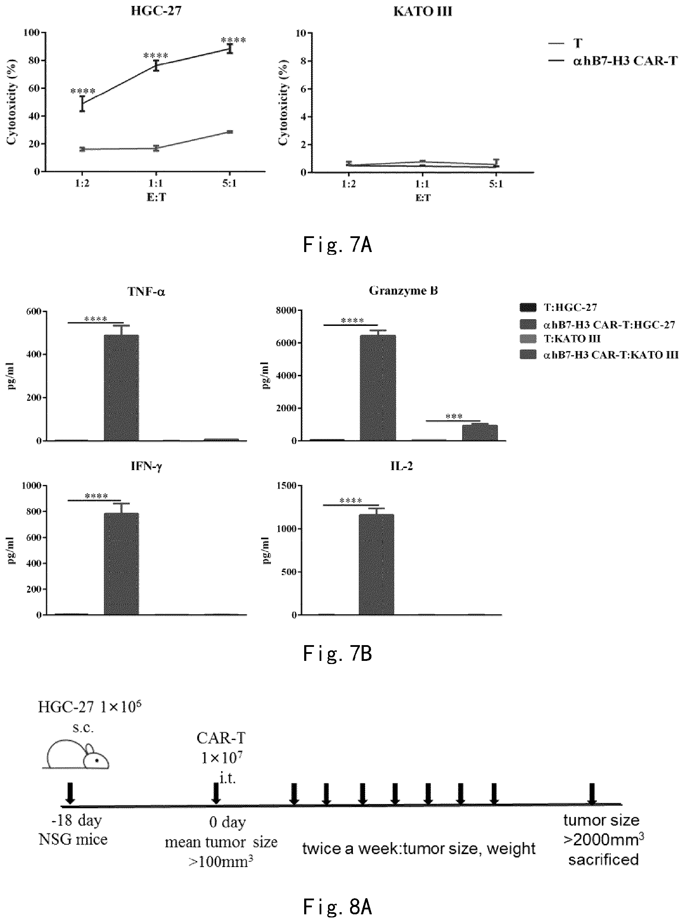

The results show that αhB7-H3 CAR-T cells have significant specific enhanced cytotoxicity against B7-H3-positive HGC-27 cells compared to T cells, while αhB7-H3 CAR-T cells have no specifically enhanced cytotoxicity against B7-H3 negative KATO-III cells (Fig. 7A). In addition, the contents of IL-2, TNF-α, IFN-γ and granzyme B in supernatant of each group in the above cytotoxicity test were also analyzed using the CBA method. The results show that after being co-cultured with B7-H3 positive tumor cells, the secretion ability of IL-2, TNF-α, IFN-γ and granzyme B of αhB7-H3 CAR-T cells is significantly stronger than that of T cells, while after being co-cultured with B7-H3 negative KOTA-III cells, the cytokine secretion of NT and B7-H3 CAR-T cells show no significant difference (Fig. 7B).

Meanwhile, the *in vitro* anti-tumor activity of the αhB7-H3 CAR-T cells was evaluated by using the RTCA method. The results show that the αhB7-H3 CAR-T cells prepared by three PBMC samples can effectively inhibit the growth of HGC-27 and AGS cells (Fig. 12A, only one group of data is shown), and the results of the supernatant cytokine detection also show that αhB7-H3 CAR-T cells have significantly enhanced cytokine secretion capacity (Fig. 12B).

In *in vivo* experiments, a xenograft tumor NSG mouse model was established by using HGC-27 cells, and the experimental process is shown in Fig. 8A. 1×10⁶ HGC-27 cells were subcutaneously injected to 7-week-old female NSG mice. The tumor size and weight were measured twice a week. When the average tumor volume reached 100 mm³, the mice were randomly divided into three groups: PBS group, T cell group, and CAR-T cell group, with 5 mice in each group. PBS with the same volume as the effector cell suspension, 1×10⁷ T cells and 1×10⁷ CAR-T cells were sequentially returned through intratumoral injection.

The results show that the tumor growth of the mice in αhB7-H3 CAR-T cell group are significantly inhibited, compared with those in the PBS group and the T cell group (Fig. 8B-D). The body weight of mice in αhB7-H3 CAR-T cell group does not significantly change during the treatment (Fig. 8E), and there is no significant difference in organ coefficients except for the liver compared to other groups of mice (Fig. 8F). In addition, blood routine detection results of the mice show that all blood routine parameters of each group of mice have no significant difference (Fig. 8G).

The above experimental results show that the αhB7-H3 CAR-T cells have good safety and effectiveness for the treatment of gastric cancer cell model mice.

### Example 8 αB7-H3 CAR-T Cells for the Treatment of Triple Negative Breast Cancer

B7-H3 antigens are highly expressed in metastatic and malignant breast cancer tissues, therefore the anti-tumor activity of αhB7-H3 CAR-T cells to triple-negative breast cancer was further evaluated.

Two strains of breast cancer cells: T47D and MDA-MB-468 were selected as target cells, wherein MDA-MB-468 is a triple negative breast cancer cell. The B7-H3 expression amount on the two breast cancer cell surface were first detected, and the flow cytometry detection results show that the two cells highly express B7-H3 antigen (Fig. 9A). Next, the inhibitory effect of αhB7-H3 CAR-T cells on the growth of two breast cancer were verified by the RTCA method. The results show that compared with T cells, the growth of two breast cancer cells can be significantly inhibited at the effect-target ratios of 1:1 and 5:1 (Fig. 9B). At the same time, the detection results of cytokine content in the supernatant in this experiment show that the secretion of IL-2, TNF-α, IFN-γ and granzyme B can be significantly enhanced after co-incubation with two strains of breast cancer cells (Fig. 9C). The above experiment results show that the αhB7-H3 CAR-T cells have significant specific cytotoxicity to breast cancer cells *in vitro.*

### Example 9 Treatment of Osteosarcoma with B7-H3 CAR-T Cells

B7-H3 molecules are highly expressed in tumor tissues of 90% or more osteosarcoma patients, and the expression of B7-H3 is closely related to the recurrence of osteosarcoma and the survival of patients. Therefore, the therapeutic effect of αhB7-H3 CAR-T cells on osteosarcoma was further evaluated.

Three strains of osteosarcoma cells, HOS, MG63 and SJSA-1, were selected for study. The B7-H3 antigen expression detection of the three cells through flow cytometry shows that the three osteosarcoma cells all have a B7-H3 antigen expression level close to 100% (Fig. 10A). The RTCA detection result of the growth inhibition of αhB7-H3 CAR-T cells on HOS cells, MG63 cells and SJSA-1 cells shows that, αhB7-H3 CAR-T cells exhibit specific inhibitory effects on the growth of the three cells under different effect-target ratios (Fig. 10B). At the same time, the detection result of the cytokine content in the supernatant in this experiment also shows that, after co-incubation with HOS cells, MG63 cells and SJSA-1 cells, the secretion amounts of IL-2, IFN-γ and granzyme B of αhB7-H3 CAR-T cells are significantly increased compared with that of T cells (Fig. 10C). The above experimental results show that the αhB7-H3 CAR-T cells have significant anti-tumor activity to osteosarcoma *in vitro.*

In *in vivo* experiments, MG63 cells are used to establish xenograft tumor mouse models, and the experimental design is shown in Fig. 11A. On day 0, 5×10⁶ MG63 cells mixed with matrigel were injected subcutaneously to the right back of NSG mice, and the tumor size and weight of mice were measured twice a week. When the average tumor volume reached 100 mm³, the mice were randomly divided into three groups: PBS group, T cell group, and αhB7-H3 CAR-T cell group, with 5 mice in each group, and each group of mice was respectively injected with PBS with the same volume as the effector cell suspension, 1×10⁷ T cells and 1×10⁷ αhB7-H3 CAR-T cells through intratumoral injection.

The results show that compared with PBS and T cells, αhB7-H3 CAR-T cells can effectively inhibit the growth of osteosarcoma cells (Fig. 11B), and at the same time, the body weight of the αhB7-H3 CAR-T cell group mice in the treatment process does not change significantly. However, in the experiment process, the weight of the T cell group mice is decreased, which may have the same reason as the weight decrease of mice in the triple negative breast cancer treatment experiment (Fig. 11C).

The above experimental results show that αhB7-H3 CAR-T cells can effectively inhibit the growth of osteosarcoma cells *in vivo* and *in vitro,* and have good safety.

### Example 10 In Vivo Antitumor Effect Of αhB7-H3 CAR-T Cells on Neuroblastoma Cell Strain

A subcutaneous tumor model of NSG mice was constructed by using a neuroblastoma cell strain SK-N-AS (B7-H3 positive cell strain), and the experimental design is shown in Fig. 13A. Each mouse was inoculated with 15×10⁶ SK-N-AS cells. A total of 10 mice were inoculated and divided into 2 groups with 5 mice in each group. Then 2×10⁷ CAR-T cells (αhB7-H3 CAR-T) or T cells (Mock-T) were respectively returned at day 7, and then the tumor size of mice and the body weight of mice were measured regularly.

The results show that αhB7-H3 CAR-T cells can significantly inhibit the growth and proliferation of tumor cells in mice (as shown in Figs. 13B and 13C) compared to the control group. In addition, the weight of the mice does not change significantly compared to the control group (as shown in Fig. 13D).

The above results show that the αhB7-H3 CAR-T cell constructed in the present invention has a significant *in vivo* anti-tumor effect on the neuroblastoma cell strain SK-N-AS.

### Example 11 In Vitro and In Vivo Anti-tumor Effect of B7-H3 CAR-T on Colon Cancer Cell Strain

A third-generation B7-H3-CAR vector containing ICOS and 41BB costimulatory molecules at the same time was constructed by using the B7-H3 humanized antibody sequence T1H1-T1L1. The structural diagram of the vector is shown in Fig. 14, and the amino acid sequence of the third-generation B7-H3-CAR is shown in SEQ ID NO: 19.

The expression of B7-H3 on the surface of colon cancer cell line SW620 cells was detected by flow cytometry, and the results in Fig. 15 showed that the positive rate of B7-H3 on SW620 cell was 95.20%, indicating that colon cancer cell line SW620 also highly expressed B7-H3 cells.

Then, SW620 was further subjected to *in vitro* killing experiments with the third-generation B7-H3-CAR-T cells, and the results as shown in Figure 16A. It shows that under the effect-target ratio of 5:1 and 1:1, compared with the control group of T cells, the third-generation B7-H3-CAR-T cells can significantly inhibit the growth of SW620 cells, and the inhibition effect on tumors under the effect-target ratio of 5:1 is more obvious than that under the effect-target ratio of 1:1. The above results indicate that the third-generation B7-H3-CAR-T cells have significant specific killing on colon cancer cell line SW620.

Then, the release of cytokines (IL-2, TNF-α, IFN-γ, GranzymeB) in the supernatant in the above experiment was further analyzed, and the result shown in Fig. 16B shows that compared with the control group, the release of the cytokine of the B7-H3-CAR-T group is significantly increased, indicating the release of specific cytokines, which further illustrates that the third-generation B7-H3-CAR-T cell constructed in the present invention can be specifically activated by SW620 cells.

In order to further evaluate the *in vivo* anti-tumor effect of the third-generation B7-H3-CAR-T cells constructed in the present invention on colon cancer, firstly tumor models of NCG mice was constructed by using Luc-GFP-SW620 cells (a SW620 cell strain over-expressing luciferase), and the experimental design is shown in Fig. 17A. On day 0, 1×10⁶ Luc-GFP-SW620 cells were infused to each mouse through the tail vein, and on day 5, the mice were randomly divided into three groups: PBS group, T cell group and CAR-T cell group. Each mouse were infused with 2×10⁷ T or CAR-T cells or equivalent PBS. Each group contains 8 mice, wherein 4 mice were only used for living body imaging and survival observation, and the other 4 mice were used for evaluating other efficacy and safety after being sacrificed and dissected. The results show that compared with the control groups of PBS and T cells, tumor fluorescence signals in mice treated with CAR-T are significantly weakened (Figs. 17B and 17C), indicating that the third-generation B7-H3-CAR-T cells can significantly inhibit the growth and proliferation of SW620 tumor cells in mice. The results of the survival curve of mice (Fig. 17D) also show that the third-generation B7-H3-CAR-T cells significantly extend the survival of SW620 tumor-bearing mice.

The above results show that the third-generation B7-H3-CAR-T cell constructed in the present invention has a significant anti-tumor effect on colon cancer cells SW620 *in vivo.*

### Example 12 In Vivo Anti-Tumor Effect of Low Dose B7-H3 CAR-T Cells Combined with Radiotherapy on Pancreatic Cancer Cells

Firstly, the expression of B7-H3 on the cell surface of the four pancreatic cancer cell strains PANC-1, SW1990, ASPC-1, and PATU8988 were detected, and the results are shown in Fig. 18A. The four pancreatic cancer cells highly express B7-H3 molecules.

Then, the *in vivo* anti-tumor effect of a low dose of the third-generation B7-H3 CAR-T cells combined with X-ray irradiation on the pancreatic cancer cells SW1990 was evaluated. 1×10⁶ SW1990 cells were used for subcutaneous tumor-bearing in NCG mice of 6-8 weeks. In a total of 12 mice, two tumor-bearing mice were taken as a control group (CONT group), 9 mice were locally irradiated with X-ray at tumor sites 8 days after tumor-bearing (as shown in Fig. 18B) with doses of 2 Gy * 1 (2 Gy irradiation once), 2 Gy * 4 (2 Gy irradiation 4 times), and 8 Gy * 1(8 Gy irradiation once). After irradiation, one mouse were taken from each irradiation dose and injected with 1×10⁶ B7-H3-CAR-T cells through tail vein. The remaining 1 unirradiated mouse was injected with 1×10⁶ B7-H3-CAR-T cells alone. The sizes of tumors in the mice were then measured by a vernier caliper.

As a result shown in Fig. 18C, the tumor sizes of mice injected with 1×10⁶ CAR-T cells alone do not change substantially compared to those of the unirradiated tumor-bearing mice; and the tumor sizes of mice irradiated locally with X-rays alone has a tendency to become smaller than those of the control group, but do not show significant change; and tumors in mice subjected to a further combination of low dose B7-H3-CAR-T cell injection and irradiation (2 Gy irradiation once, 2 Gy irradiation for 4 times, and 8 Gy irradiation once) show a distinct tendency to become smaller, especially the combination therapy group of 8 Gy irradiation once, wherein substantially no tumors can be detected at day 30. It shows that low dose B7-H3 CAR-T cells combined with radiotherapy can further improve the *in vivo* anti-tumor effect on pancreatic cancer cells SW1990.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

Sequences involved in the sequence listing of this application are as follows:

| SEQ ID NO | Sequence Name | Sequence |
|---|---|---|
| 1 | Murine VH | EVTLVESGGGLVKPGGSLKLSCAASGFTFSTYVMSWVRQTP EKRLEWVASILSGGTSFYADNVKGRFTISRENARNILYLQMS SLKSEDTAMYYCVRRYGHDHYYAMDYWGQGTSVTVSS |
| 2 | VH CDR1 | GFTFSTY |
| 3 | VH CDR2 | LSGGT |
| 4 | VH CDR3 | RYGHDHYYAMDY |
| 5 | Murine VL | QIVLTQSPANMSASLGERVTMTCAASSSVSSSYLHWYQQKP GSSPKLWIYSTSNLASGVPGRFNGSGSGTSYSLTISSMEAED AATYYCHQYHRSPWTFGGGTKLEIK |
| 6 | VL CDR1 | AASSSVSSSYLH |
| 7 | VL CDR2 | STSNLAS |
| 8 | VL CDR3 | QYHRSPWT |
| 9 | Humanized VH (T1H1) | EVQLVESGGGLIQPGGSLRLSCAASGFTFSTYVMSWVRQAP GKGLEWVSSILSGGTSFYADNVKGRFTISRDNSKNTLYLQM NSLRAEDTAVYYCARRYGHDHYYAMDYWGQGTLVTVSS |
| 10 | Humanized VL (T1L1) | EIVLTQSPATLSLSPGERATLSCAASSSVSSSYLHWYQQKPG LAPRLLIYSTSNLASGIPDRFSGSGSGTDFTLTISRLEPEDFAV YYCQQYHRSPWTFGGGTKVEIK |
| 11 | Humanized VH (T2H1) | EVQLVESGGGVVRPGGSLRLSCAASGFTFSTYVMSWVRQA PGKGLEWVSSILSGGTSFYADNVKGRFTISRDNAKNSLYLQ MNSLRAEDTALYHCARRYGHDHYYAMDYWGQGTTVTVSS |
| 12 | Humanized VL (T2L1) | DIQMTQSPSTLSASVGDRVTITCAASSSVSSSYLHWYQQKP GKAPKLLIYSTSNLASGVPSRFSGSGSGTEFTLTISSLQPDDF ATYYCQQYHRSPWTFGQGTKLEIK |
| 13 | Humanized scFv (T1H1-T1L1 ) | EVQLVESGGGLIQPGGSLRLSCAASGFTFSTYVMSWVRQAP GKGLEWVSSILSGGTSFYADNVKGRFTISRDNSKNTLYLQM NSLRAEDTAVYYCARRYGHDHYYAMDYWGQGTLVTVSSG GGGSGGGGSGGGGSGGGGSEIVLTQSPATLSLSPGERATLSC AASSSVSSSYLHWYQQKPGLAPRLLIYSTSNLASGIPDRFSG SGSGTDFTLTISRLEPEDFAVYYCQQYHRSPWTFGGGTKVEI K |
| 14 | Humanized scFv (T2H1-T2L1 ) | EVQLVESGGGVVRPGGSLRLSCAASGFTFSTYVMSWVRQA PGKGLEWVSSILSGGTSFYADNVKGRFTISRDNAKNSLYLQ MNSLRAEDTALYHCARRYGHDHYYAMDYWGQGTTVTVSS GGGGSGGGGSGGGGSGGGGSDIQMTQSPSTLSASVGDRVTI TCAASSSVSSSYLHWYQQKPGKAPKLLIYSTSNLASGVPSR FSGSGSGTEFTLTISSLQPDDFATYYCQQYHRSPWTFGQGTK LEIK |
| 15 | tEGFR | RKVCNGIGIGEFKDSLSINATNIKHFKNCTSISGDLHILPVAF RGDSFTHTPPLDPQELDILKTVKEITGFLLIQAWPENRTDLH AFENLEIIRGRTKQHGQFSLAVVSLNITSLGLRSLKEISDGDV IISGNKNLCYANTINWKKLFGTSGQKTKIISNRGENSCKATG QVCHALCSPEGCWGPEPRDCVSCRNVSRGRECVDKCNLLE GEPREFVENSECIQCHPECLPQAMNITCTGRGPDNCIQCAH YIDGPHCVKTCPAGVMGENNTLVWKYADAGHVCHLCHPN CTYGCTGPGLEGCPTNGPKIPSIATGMVGALLLLLVVALGIG LFM |
| 16 | αhB7-H3 CAR | MALPVTALLLPLALLLHAARPEVQLVESGGGLIQPGGSLRL SCAASGFTFSTYVMSWVRQAPGKGLEWVSSILSGGTSFYAD NVKGRFTISRDNSKNTLYLQMNSLRAEDTAVYYCARRYGH DHYYAMDYWGQGTLVTVSSGGGGSGGGGSGGGGSGGGG SEIVLTQSPATLSLSPGERATLSCAASSSVSSSYLHWYQQKPG LAPRLLIYSTSNLASGIPDRFSGSGSGTDFTLTISRLEPEDFAV YYCQQYHRSPWTFGGGTKVEIKTTTPAPRPPTPAPTIASQPL SLRPEACRPAAGGAVHTRGLDFACDIYIWAPLAGTCGVLLL SLVITLYCKRGRKKLLYIFKQPFMRPVQTTQEEDGCSCRFPE EEEGGCELRVKFSRSADAPAYKQGQNQLYNELNLGRREEY DVLDKRRGRDPEMGGKPRRKNPQEGLYNELQKDKMAEAY SEIGMKGERRRGKGHDGLYQGLSTATKDTYDALHMQALPP R |
| 17 | Linking peptide fragment | GGGGS |
| 18 | Murine scFv (mB7-H3) | EVTLVESGGGLVKPGGSLKLSCAASGFTFSTYVMSWVRQTP EKRLEWVASILSGGTSFYADNVKGRFTISRENARNILYLQMS SLKSEDTAMYYCVRRYGHDHYYAMDYWGQGTSVTVSSG GGGSGGGGSGGGGSGGGGSQIVLTQSPANMSASLGERVTM TCAASSSVSSSYLHWYQQKPGSSPKLWIYSTSNLASGVPGR FNGSGSGTSYSLTISSMEAEDAATYYCHQYHRSPWTFGGGT KLEIK |
| 19 | Third-generat ion B7-H3-CAR | MALPVTALLLPLALLLHAARPEVQLVESGGGLIQPGGSLRLSCAASG FTFSTYVMSWVRQAPGKGLEWVSSILSGGTSFYADNVKGRFTISRDN SKNTLYLQMNSLRAEDTAVYYCARRYGHDHYYAMDYWGQGTLVT VSSGGGGSGGGGSGGGGSGGGGSEIVLTQSPATLSLSPGERATLSCAA SSSVSSSYLHWYQQKPGLAPRLLIYSTSNLASGIPDRFSGSGSGTDFTL TISRLEPEDFAVYYCQQYHRSPWTFGGGTKVEIKESKYGPPCPPCPAP EFEGGPSVFLFPPKPKDTLMISRTPEVTCVVVDVSQEDPEVQFNWYV DGVEVHNAKTKPREEQFQSTYRVVSVLTVLHQDWLNGKEYKCKVS NKGLPSSIEKTISKAKGQPREPQVYTLPPSQEEMTKNQVSLTCLVKGF YPSDIAVEWESNGQPENNYKTTPPVLDSDGSFFLYSRLTVDKSRWQE GNVFSCSVMHEALHNHYTQKSLSLSLGKWLPIGCAAFVVVCILGCIL ICWLTKKKYSSSVHDPNGEYMFMRAVNTAKKSRLTDVTLKRGRKKL LYIFKQPFMRPVQTTQEEDGCSCRFPEEEEGGCELRVKFSRSADAPAY KQGQNQLYNELNLGRREEYDVLDKRRGRDPEMGGKPRRKNPQEGL YNELQKDKMAEAYSEIGMKGERRRGKGHDGLYQGLSTATKDTYDA LHMQALPPR |

## Claims

1. An antibody targeting B7-H3, which comprises a heavy chain variable region and/or a light chain variable region;
wherein the heavy chain variable region comprises the following complementarity determining regions (CDRs):
a CDR1 shown in SEQ ID NO: 2;
a CDR2 shown in SEQ ID NO: 3; and
a CDR3 shown in SEQ ID NO: 4;
the light chain variable region comprises the following complementarity determining regions (CDRs):
a CDR1' shown in SEQ ID NO: 6;
a CDR2' shown in SEQ ID NO: 7; and
a CDR3' shown in SEQ ID NO: 8.

2. The antibody according to claim 1, wherein the amino acid sequence of the heavy chain variable region of the antibody is shown in SEQ ID NO: 1, 9 or 11; and/or
the amino acid sequence of the light chain variable region of the antibody is shown in SEQ ID NO: 5, 10 or 12.

3. A scFv targeting B7-H3, wherein the heavy chain variable region of the scFv comprises the following complementarity determining regions (CDRs):
a CDR1 shown in SEQ ID NO: 2;
a CDR2 shown in SEQ ID NO: 3; and
a CDR3 shown in SEQ ID NO: 4; and
the light chain variable region of the scFv comprises the following complementarity determining regions (CDRs):
a CDR1' shown in SEQ ID NO: 6;
a CDR2' shown in SEQ ID NO: 7; and
a CDR3' shown in SEQ ID NO: 8.

4. A chimeric antigen receptor comprising the scFv according to claim 3.

5. A polynucleotide encoding a polypeptide selected from the group consisting of:
(1) the antibody according to claim 1;
(2) the scFv according to claim 3; and
(3) the chimeric antigen receptor according to claim 4.

6. A vector comprising the polynucleotide according to claim 5.

7. A genetically engineered host cell, which comprises the vector according to claim 6, or has the exogenous polynucleotide according to claim 5 integrated into the genome, or expresses the antibody according to claim 1, or expresses the scFv according to claim 3, or expresses the chimeric antigen receptor according to claim 4.

8. An antibody conjugate comprising:
(a) an antibody moiety, which is selected from the group consisting of the antibody according to claim 1; and
(b) a coupling moiety coupled to the antibody moiety, which is selected from the group consisting of a detectable label, a drug, a toxin, a cytokine, a radionuclide, an enzyme, and a combination thereof.

9. A method for preparing an engineered cell expressing the antibody according to claim 1, the scFv according to claim 3 or the chimeric antigen receptor according to claim 4, which comprises a step of: transducing the polynucleotide according to claim 5 or the vector according to claim 6 into a host cell, thereby obtaining the engineered cell.

10. Use of an active ingredient selected from the group consisting of: the antibody according to claim 1, the scFv according to claim 3, the chimeric antigen receptor according to claim 4, the vector according to claim 6, the host cell according to claim 7, or the antibody conjugate according to claim 8, for (a) preparation of a diagnostic reagent or kit; and/or (b) preparation of a medicament or formulation for the prevention and/or treatment of a tumor or cancer.
